# EUROPEAN PATENT APPLICATION

(11) **EP 4 299 119 A1**
(43) Date of publication of application: **03.01.2024**
(21) Application number: 22182000.4
(22) Date of filing: 29.06.2022
(51) Int. Cl.: A61P 9/10, A61P 35/00, C07K 14/715, G01N 33/50

(54) **THERAPEUTIC APPLICATIONS BASED ON THE INHIBITION OF G PROTEIN-COUPLED RECEPTOR 182 (GPR182)**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to an agent that inhibits the expression or activity of the G Protein-Coupled Receptor 182 (GPR182) protein for use in the treatment or prevention of a pathological condition selected from myocardial infarction, myocardial ischemia, myocardial necrosis, cardiac hypertrophy, cardiac fibrosis, limb ischemia, ischemia-related tissue degeneration, stroke and a cancer, wherein (a) the agent that inhibits the expression of the GPR182 protein is selected from an siRNA, miRNA, shRNA, a ribozyme and an antisense nucleic acid molecule; and/or (b) the agent that inhibits the activity of the GPR182 protein specifically binds to the GPR182 protein and inhibits binding of one or more endogenous ligands to the GPR182 protein, and wherein the agent is selected from an antibody, an Fc fusion polypeptide, an adnectin, an affibody, an affilin, an anticalin, an atrimer, an avimer, an evibody, a Kunitz-type domain, a designed ankyrin repeat protein (DARPin), a fynomer, a peptide or peptidomimetic, an aptamer, and a small molecule; or any combination and/or hetero- or homo-oligomeric, covalent or non-covalent complex thereof.

## Description

The present invention relates to an agent that inhibits the expression or activity of the G Protein-Coupled Receptor 182 (GPR182) protein for use in the treatment or prevention of a pathological condition selected from myocardial infarction, myocardial ischemia, myocardial necrosis, cardiac hypertrophy, cardiac fibrosis, limb ischemia, ischemia-related tissue degeneration, stroke and a cancer, wherein (a) the agent that inhibits the expression of the GPR182 protein is selected from an siRNA, miRNA, shRNA, a ribozyme and an antisense nucleic acid molecule; and/or (b) the agent that inhibits the activity of the GPR182 protein specifically binds to the GPR182 protein and inhibits binding of one or more endogenous ligands to the GPR182 protein, and wherein the agent is selected from an antibody, an Fc fusion polypeptide, an adnectin, an affibody, an affilin, an anticalin, an atrimer, an avimer, an evibody, a Kunitz-type domain, a designed ankyrin repeat protein (DARPin), a fynomer, a peptide or peptidomimetic, an aptamer, and a small molecule; or any combination and/or hetero- or homo-oligomeric, covalent or non-covalent complex thereof.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Myocardial infarction (MI) is one of the most common acute diseases and causes of death worldwide. It is caused by acute, usually thrombotic occlusion of a coronary artery, resulting in ischemic myocardial necrosis. Because of the incapability of postnatal cardiomyocytes to re-enter the cell cycle and proliferate, in adult mammals, the dead cells are replaced with a permanent collagenous scar instead of new cardiac muscle tissue. This also induces geometrical, biomechanical, and biochemical changes in the uninjured ventricular wall eliciting a reactive remodeling process that includes interstitial and perivascular fibrosis. Although the initial reparative fibrosis is crucial for preventing rupture of the ventricular wall, an exaggerated fibrotic response and reactive fibrosis outside the injured area are detrimental as they lead to progressive impairment of cardiac function and eventually to heart failure (Talman V, Ruskoaho H. Cell Tissue Res. 2016;365(3):563-581). In addition to early lethal complications such as cardiac arrhythmias, acute heart failure, and cardiogenic shock, late complications (i.e., so-called post-complications of MI) such as chronic heart failure, heart wall aneurysms, and arrhythmias play a central role in reducing quality of life as well as life expectancy after myocardial infarction in patients who survive the acute post-infarction period. Any therapeutic measure that reduces infarct size has a favorable effect on short- and long-term prognosis.

Current therapies of acute MI are largely based on reversing the effects of ischemia/reperfusion injury by promoting reperfusion. These measures involve the administration of fibrinolytics such as streptokinase or tissue plasminogen activator (tPA) variants. In parallel, anticoagulants and platelet function inhibitors are used to suppress further thrombus formation. Alternatively, or in parallel, percutaneous transluminal coronary angioplasty with balloon catheter dilatation and/or stent implantation are/is used as part of reperfusion therapy. Reperfusion therapy is further supplemented by various symptomatic measures, such as O₂ supply and administration of nitrates, beta blockers and/or angiotensin-converting enzyme (ACE) inhibitors, partly to improve hemodynamic parameters. However, despite the availability of these current therapeutic options, the acute lethality of MI is still too high (3-9%), and a large proportion of patients after acute MI suffers from a poor long-term prognosis with severely reduced life expectancy (Zeymer, 2019). Thus, these therapies, although to a certain extent effective in ameliorating MI symptoms, do not result in a fully functional recovery of the affected tissue. Although more recent experimental preclinical approaches have focused on enhancing cardioprotection by increasing ischemia tolerance, promoting the regenerative potential, and inducing vascular growth, no therapeutic approach has yet been identified to effectively prevent or reduce myocardial scar tissue formation and/or replacement thereof with functioning contractile tissue after myocardial infarction.

Cancer is another leading cause of death worldwide, with an estimated 12.7 million cases around the world affecting both sexes equally. This number is expected to even increase to 21 million by 2030. Whereas earlier cancer treatments relied upon surgical intervention, radiotherapy and/or chemotherapy, an increasing number of novel anti-cancer treatments have meanwhile become available which are aimed at activating the patient's own immune cells to attack cancer. However, a major hurdle to the effectiveness of so-called "cancer immunotherapies" (also commonly referred to as "immune-oncology") is that many cancers are able to circumvent the immune recognition based on their capacity to undergo immune evasion, for example, by expression of ligands on their surfaces which inhibit immune cell recruitment and/or activity and thus their ability to combat cancer. In the past decades, novel cancer treatment strategies based on adoptive cellular therapies esp. CAR-T cell therapies have emerged which, by expressing chimeric antigen receptors (CARs) on their surfaces, can redirect a cancer patient's own or a donor's immune cells to tumor-specific surface antigens. In addition, so-called immune checkpoint inhibitors (ICIs) have emerged as a novel class of immunotherapy drugs. These humanized monoclonal antibodies target inhibitory receptors (e.g., CTLA-4, PD-1, LAG-3, TIM-3) and ligands (PD-L1) expressed on immune cells (e.g., T lymphocytes, antigen presenting cells) or tumor cells and thereby elicit an anti-tumor response by stimulating the immune system (see, for example, Vinay DS *et al.,* Semin Cancer Biol. 2015 Dec;35 Suppl:S185-S1). However, despite of these advances, the current understanding of the mechanisms underlying cancer immune evasion is only limited, and therapeutic outcomes from available cancer immunotherapies are often poor.

Thus, there is a need for alternative, more effective therapeutic strategies for the treatment and prevention of these and related conditions and post-complications thereof. The present invention addresses this need and several further aspects as demonstrated in the appended examples.

Accordingly, the present invention relates in a first aspect to an agent that inhibits the expression or activity of the G Protein-Coupled Receptor 182 (GPR182) protein for use in the treatment or prevention of a pathological condition selected from myocardial infarction, myocardial ischemia, myocardial necrosis, cardiac hypertrophy, cardiac fibrosis, limb ischemia, ischemia-related tissue degeneration, stroke and a cancer, wherein (a) the agent that inhibits the expression of the GPR182 protein is selected from an siRNA, an miRNA, an shRNA, a ribozyme and an antisense nucleic acid molecule; and/or (b) the agent that inhibits the activity of the GPR182 protein specifically binds to the GPR182 protein and inhibits binding of one or more endogenous ligands to the GPR182 protein, and wherein the agent is selected from an antibody, an Fc fusion polypeptide, an adnectin, an affibody, an affilin, an anticalin, an atrimer, an avimer, an evibody, a Kunitz-type domain, a designed ankyrin repeat protein (DARPin), a fynomer, a peptide or peptidomimetic, an aptamer, and a small molecule; or any combination and/or hetero- or homo-oligomeric, covalent or non-covalent complex thereof.

G protein-coupled receptors (GPCRs) represent the largest group of transmembrane receptors encoded in the genome, and they are the largest group of proteins targeted by approved drugs. GPCRs are very versatile and can bind ligands of different physicochemical properties, including ions, lipids, biogenic amines, peptides, or proteins, such as chemokines. Primarily by activation of heterotrimeric G proteins, typical GPCRs regulate multiple functions in basically all cells of mammalian organisms. Despite their large physiological and pharmacological relevance, the endogenous ligands, activating mechanisms and physiological functions of more than 100 GPCRs are still not known and these receptors are therefore referred to as "orphan" receptors.

The "G Protein-Coupled Receptor 182 (GPR182)", formerly known as adrenomedullin receptor (ADMR), is a member of the "Class A" G-protein coupled receptor (GPCR) family. Whereas GPR182 was previously found to be expressed on endothelial cells, its ligand(s) and physiological role have remained elusive. However, in a recent study *inter alia* by the present inventors (Le Mercier A et al. Proc Natl Acad Sci USA. 2021;118(17):e2021596118), GPR182 was revealed to be expressed in microvascular and lymphatic endothelial cells of most organs, and to bind with nanomolar affinity the chemokine ligands CXCL10, CXCL12, and CXCL13. In contrast to conventional chemokine receptors, however, binding of these ligands to GPR182 was found to not induce typical downstream signaling processes, such as G_{q}- and Gᵢ-mediated signaling or β-arrestin recruitment. Instead, GPR182 was identified to have relatively high constitutive activity with respect to β-arrestin recruitment and to rapidly undergo internalization in a ligand-independent manner. Further exploration based on a GPR182-deficient mouse model revealed that the absence of GPR182 expression causes significant increases of the plasma levels of its chemokine ligands CXCL10, CXCL12, and CXCL13. Based on these findings and the observed absence of typical signaling functions, GPR182 is presumed to act as a scavenging receptor controlling the plasma levels of its chemokine ligands via their receptor-internalization-mediated removal.

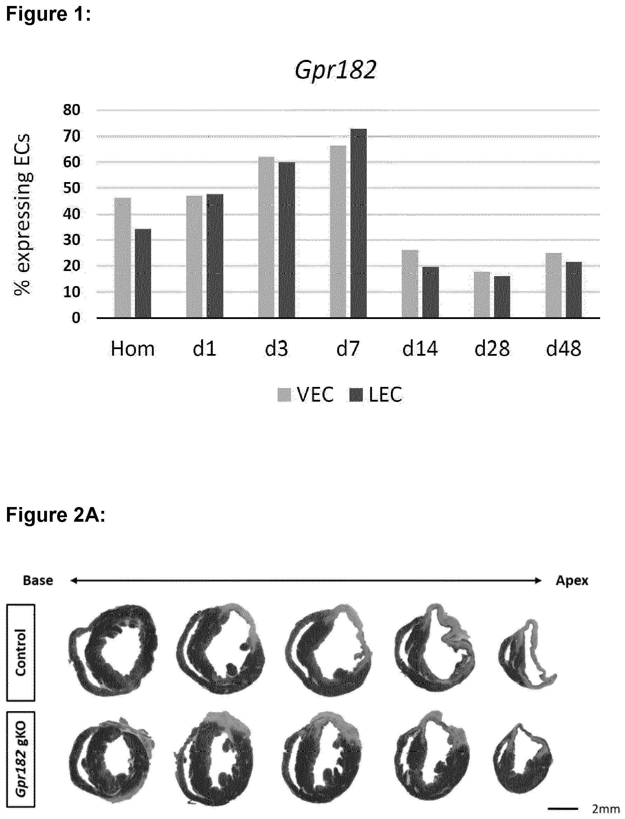

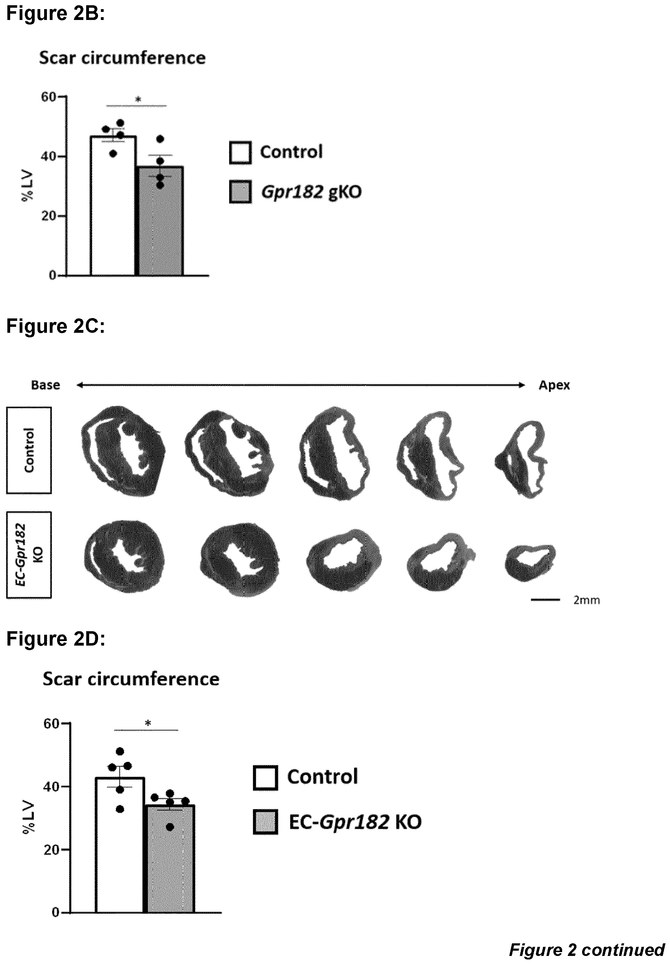

Not excluding that an inhibition of the GPR182-chemokine axis may create new vulnerabilities for potential therapeutic exploitation, the inventors conducted further research exploring potential implications of GPR182 in the pathology and recovery of myocardial infarction (MI). As reported in the herein disclosed examples, the inventors assessed the possibility of any alterations of GPR182-expression in heart tissue in consequence of acute myocardial infarction (MI), and further explored the possibility of any effect(s) which may result from inhibiting the GPR182/chemokine ligand axis in a murine model of acute myocardial infarction (MI). As a result of these investigations, it was found by the inventors that mice with either global or inducible endothelium-specific loss of the GPR182 showed significantly increased levels of the chemokine CXCL12 in their infarcted heart. Moreover, it was surprisingly and advantageously found that the mice lacking GPR182 expression showed markedly reduced infarct sizes (which is known to be directly correlated with MI mortality) as well as improved hemodynamic parameters, including an increased ejection fraction of the left ventricle as well as reduced left ventricular end-diastolic and end-systolic volumes. These observations (see, e.g., Example 1, and Figs. 2 to 4) suggest a strong therapeutic potential for inhibiting the GPR182-mediated scavenging function by agents either inhibiting the expression or the activity of the GPR182 protein (esp. agents blocking the binding of one or more of its chemokine ligands (e.g., CXCL12)) for the treatment of (acute) myocardial infarction and post-complications thereof (esp. for improving post-acute recovery and thus the long-term prognosis), as well as related further pathological conditions.

Particularly pathological conditions that are envisaged for being prevented and/or treated by the medical uses of the present invention include myocardial infarction (and any known acute-phase or post-complication thereof), myocardial ischemia, myocardial necrosis, cardiac hypertrophy, cardiac fibrosis, limb ischemia, ischemia-related tissue degeneration, stroke and/or a cancer.

As used herein, the term "myocardial infarction (MI)", also commonly known as "heart attack", refers to the irreversible tissue death (infarction) of the heart muscle (myocardium) caused by ischemia, the lack of oxygen delivery to myocardial tissue. It is a type of acute coronary syndrome, which describes a sudden or short-term change in symptoms related to blood flow to the heart. Unlike the other type of acute coronary syndrome, unstable angina, a myocardial infarction (MI) occurs when there is cell death, which can be estimated by a blood test for biomarkers (e.g., the cardiac protein troponin). When there is evidence of an MI, it may be classified as an ST elevation myocardial infarction (STEMI) or Non-ST elevation myocardial infarction (NSTEMI) based on the results of an ECG. An MI is different from - but can cause - cardiac arrest, where the heart is not contracting at all or so poorly that all vital organs cease to function, thus might lead to death. It is also distinct from heart failure, in which the pumping action of the heart is impaired. However, an MI may lead to heart failure. The term "MI" as used herein also refers to so-called "silent myocardial infarctions" which can happen without any symptoms at all. These cases can be discovered later on electrocardiograms, using blood enzyme tests, or at autopsy after a person has died. Such silent myocardial infarctions represent between 22 and 64% of all infarctions, and are more common in the elderly, in those with diabetes mellitus and after heart transplantation. In people with diabetes, differences in pain threshold, autonomic neuropathy, and psychological factors have been cited as possible explanations for the lack of symptoms. In heart transplantation, the donor heart is not fully innervated by the nervous system of the recipient. After MI, extensive remodelling of the extracellular matrix contributes to scar formation. While aiming to preserve tissue integrity, this fibrotic response is also associated with adverse events, including a markedly increased risk of heart failure, ventricular arrhythmias, and sudden cardiac death. Cardiac fibrosis is characterized by extensive deposition of collagen and also by increased stiffness as a consequence of enhanced collagen cross-linking. Known (acute-phase and/or post-) complications of MI include, without intended to be limiting, sudden death, arrhythmia, cardiac failure (including acute and chronic heart failure), cardiogenic shock, heart wall aneurysm, ventricular rupture, rupture of papillary muscles with acute valve failure and mural thrombus with potential for thromboembolisation, myocardial ischemia, myocardial necrosis, cardiac fibrosis, cardiac hypertrophy, and heart insufficiency.

As used herein, the term "ischemia" or "ischaemia" refers to a restriction in blood supply to any tissues, muscle group, or organ of the body, causing a shortage of oxygen that is needed for cellular metabolism (to keep tissue alive). Ischemia is generally caused by problems with blood vessels, with resultant damage to or dysfunction of tissue, i.e., hypoxia and microvascular dysfunction. It also means local hypoxia in a given part of a body usually resulting from reduced or interrupted blood flow (due to, e.g., vasoconstriction, thrombosis or embolism). Ischemia comprises not only insufficiency of oxygen, but also reduced availability of nutrients and inadequate removal of metabolic wastes. Ischemia can be partial (poor perfusion) or total blockage.

As used herein, the term "myocardial ischemia" or "cardiac ischemia" refers to a disorder of cardiac function caused by insufficient blood flow to the muscle tissue of the heart. The decreased blood flow may, for example, be due to narrowing of the coronary arteries (coronary atherosclerosis), due to obstruction by a thrombus (coronary thrombosis), or less commonly, due to diffuse narrowing of arterioles and other small vessels within the heart. Severe interruption of the blood supply to the myocardial tissue may result in necrosis of cardiac muscle (myocardial infarction).

As used herein, the term "myocardial necrosis" refers to any myocardial cell death regardless of its cause. Although MI is one cause of myocardial necrosis, many other conditions result in necrosis. The agents, compositions and methods of the invention can be used to promote angiogenesis and thus to reduce the blood and oxygen deprivation caused by ischemia. Such agents, compositions and methods are hence useful in reducing myocardial damage following myocardial infarction, and thus in preventing or reducing the extent of myocardial necrosis.

As used herein, the term "cardiac hypertrophy" refers to the process in which adult cardiac myocytes respond to stress through hypertrophic growth. Such growth is characterized by cell size increases without cell division, assembling of additional sarcomeres within the cell to maximize force generation, and an activation of a fetal cardiac gene program. Cardiac hypertrophy is often associated with increased risk of morbidity and mortality. Moreover, cardiac hypertrophy is a condition that can follow myocardial infarction as a process for compensation of damaged myocardium and preservation of cardiac function (see, e.g., Rubin SA. J Am Coll Cardiol. 1983;1(6):1435-41).

"Cardiac fibrosis", a common pathophysiologic process in most heart diseases such as MI, hypertensive heart disease, and different types of cardiomyopathies, refers to an excess of extracellular matrix (ECM) deposition by cardiac fibroblasts (CFs), which can lead to cardiac dysfunction and ultimately to heart failure. Taking MI as an example, sudden massive loss of cardiomyocytes triggers an intense inflammation and causes the dead myocardium to be replaced with a collagen-based scar, which is critical to prevent cardiac rupture. However, prolonged or excessive fibrotic responses can lead to excessive ECM deposition, which results in hardening of myocardium, poor tissue compliance, and worsening of cardiac dysfunction. According to the location of cardiac scars and underlying cause, cardiac fibrosis can be classified into various forms, among which reactive interstitial fibrosis and replacement fibrosis are the most relevant types of the ischemic adult heart (see, for example, Jiang W, et. al. Front Cardiovasc Med. 2021 Aug 16;8:715258).

The term "limb ischemia" (LI), as used herein, relates to a restriction in the blood supply to the limbs, generally due to factors in the blood vessels, with resultant damage or dysfunction of tissue. Limb ischemia is also known as a manifestation of peripheral arterial disease characterised by intractable pain and tissue gangrene and may result from a number of factors, including atherosclerosis. Through limb ischemia, peripheral arterial disorders, atherosclerotic peripheral vascular disease, and embolic occlusion can directly arise by reducing the blood perfusion into the limb tissues. Pain in the ischemic regions, thickening of the toenails, skin infections, and limb ulcers are considered the main symptoms of LI. Interventions such as vascular and endovascular surgery are commonly used as standard approaches to help regulate and promote circulation to ischemic limbs. Despite prescribed treatments, a significant number of patients with LIs have to undergo a major lower limb amputation (see, e.g., Khodayari S et al. Front Cell Dev Biol. 2022 May;10:834754). Hence, the development of a safe, minimally invasive, and effective strategy for regenerating degenerated tissues is regarded as the primary strategy for the treatment of the LI.

As used herein, the term "ischemia-related tissue degeneration" refers broadly to any tissue degeneration processes, for example tissue necrosis (e.g., cardiac necrosis in MI), resulting from an ischemia/reperfusion (IR) insult.

CXCL12 has previously been identified as a key positive regulator of tissue repair after stroke by its interaction with the G-protein coupled receptor CXCR4 (Wang Y et al., Curr Drug Targets. 2012 Feb;13(2):166-72). As GPR182 has also been found to be expressed in brain endothelial cells, it is considered plausible that an inhibition of the GPR182/CXCL12 interaction as envisaged herein and the resulting increase of available CXCL12 for interacting with CXCR4 will provide a therapeutic benefit for the treatment of stroke, *inter alia*, by promoting/enhancing the tissue regeneration.

The term "stroke" as used herein means an occurrence of a disturbance in the supply of blood to a mammalian subject's brain that results in rapid loss of brain functions exemplified by an inability to move one or more limbs and/or inability to perceive sensations and/or an inability to understand or formulate speech and/or loss of sight in one or both eyes. The neurological damage caused by the disturbance in supply of blood to the brain may be temporary or permanent, depending on the severity of the disturbance and on how quickly medical intervention was provided.

The term "ischemic stroke" as used herein means a stroke resulting from a lack of blood flow to the brain caused by a blockage(s) in the vascular system supplying the brain. The blockages may be due to one or more of a thrombosis, an embolism, and/or a systemic hypoperfusion. The term "hemorrhagic stroke" as used herein means a stroke resulting from a rapid accumulation of blood within or about the brain within the skull whereby the increased pressure exerted by the accumulated blood interferes with blood flow to and through the brain.

Moreover, a most recent study by Torphy *et al.* revealed that GPR182 also acts as a scavenging receptor for the chemokines CXCL9, CXCL10 and CXCL11. The latter chemokines have previously been identified to play crucial roles in anti-tumor immunity by regulating immune cell homing into tumors through their interaction with chemokine receptors expressed on immune cells. More specifically, the chemokines CXCL9-11 were found to be produced at tumor sites and, by attaching to glycosaminoglycans (GAGs) on endothelial cells (ECs) and in the extracellular matrix, to create gradients that guide T cells into the tumor or tumor microenvironment (TME). Torphy *et al.* found GPR182 to be upregulated in tumor-associated endothelial cells, in that specific case on lymphatic endothelial cells within melanoma, and, by scavenging chemokine ligands (esp. CXCL9-11), to limit effector T cell infiltration and associated antitumor immunity. In addition, an ablation of GPR182 was found to attenuate tumor growth by causing increased intratumoral chemokine levels which triggered an increase in infiltration of effector CD4+ and CD8+ T cells. It was therefore proposed by the authors that a GPR182 ablation sensitizes tumors to immunotherapy (Torphy RJ, et al. Nat Commun. 2022 Jan 10;13(1):97). The present inventors, in view of their further findings, consider it plausible that an inhibition of the expression and/or activity of GPR182 as contemplated herein will also provide effective means for enhancing antitumor immunity, specifically in melanoma and other cancers/tumors with lymphatic involvement. Moreover, it is thought that a corresponding inhibition of GPR182 by agents contemplated herein will also provide effective means to sensitizing poorly immunogenic tumors to immune checkpoint blockade and adoptive cellular therapies.

The term "cancer", as used herein, includes any malignant tumor including, but not limited to, carcinoma and sarcoma. Cancer arises from the uncontrolled and/or abnormal division of cells that then invade and destroy the surrounding tissues. As used herein, "proliferating" and "proliferation" refer to cells undergoing mitosis. As used herein, "metastasis" refers to the distant spread of a malignant tumor from its sight of origin. Cancer cells may metastasize through the bloodstream, through the lymphatic system, across body cavities, or any combination thereof. The term "cancerous cell" as provided herein, includes a cell afflicted by any one of the cancerous conditions provided herein. The term "carcinoma" refers to a malignant new growth made up of epithelial cells tending to infiltrate surrounding tissues, and to give rise to metastases.

As used herein, the term "tumor microenvironment" (TME) or "tumor stroma" refers to the non-cancer cell and non-immune cell components of tumors and is viewed traditionally as the structural components holding tumor tissues together. Tumor stroma is composed of extracellular matrix and specialized connective tissue cells, including fibroblasts and mesenchymal stromal cells. All tumors have stroma and require stroma for nutritional support and the removal of waste products.

In view of the herein contemplated purposes, it is particularly preferred that the cancer is one which comprises cells that express the GPR182 protein (i.e., a GPR182-positive cancer). In such cancers, an inhibition of the expression and/or activity of the GPR182 protein will be particularly effective to increase the chemokine ligand levels (in particular of CXCL9, CXCL10, CXCL11, and/or CXCL12) within the tumor and/or the tumor microenvironment and to thereby enhance the anti-tumor immunity by promoting the chemokine-mediated recruitment of tumor-infiltrating immune cells (esp. effector T cells) (see Torphy *et al.,* 2022). Various cancers are known to express GPR182 protein. For example, GPR182 was most recently identified to be upregulated in lymphatic endothelial cells (LECs) within human melanoma.

Thus, in preferred embodiments, the cancer is (*i*) a tumor that comprises, and/or which TME comprises lymphatic endothelial cells (LECs) and/or (*ii*) a tumor that is poorly immunogenic, preferably due to GPR182 expression by tumor cells and/or the tumor microenvironment.

In further preferred embodiments, the cancer is a cancer with a lymphatic involvement. As used herein, the term "cancer with a lymphatic involvement", interchangeably referred to herein as a "cancer with tumor-associated lymphatic vessels", means such cancers which have spread/metastasize through the lymphatic system to distant sites, e.g., lymph nodes (i.e., involving a process also known as "lymphatic metastasis"). Known cancers with lymphatic involvement include, for example, without intended to being limiting, melanoma, colorectal cancer, and breast cancer.

In particularly preferred embodiments, the cancer is a melanoma. The term "melanoma", as used herein, refers generally to a malignant tumor of melanocytes which are found predominantly in skin but also in bowel and the eye. "Melanocytes" refer to cells located in the bottom layer, the basal lamina, of the skin's epidermis and in the middle layer of the eye. Thus, "melanoma metastasis" refers to the spread of melanoma cells to regional lymph nodes and/or distant organs (e.g., liver, brain, breast, prostate, etc.).

In alternative preferred embodiments, the cancer is a lymphatic cancer. The term "lymphatic cancer", as interchangeably used herein with the term "lymphatic tumor" or "lymphoma", in distinction to a "cancer with lymphatic involvement", refers to such cancers which development starts in the lymphatic system from cancerous white blood cells. Known lymphatic cancers include, for example, without intended to being limiting, blood and lymphoid tumors (e.g., Hodgkin's disease, Non-Hodgkin's lymphoma, Burkitt's lymphoma, AIDS-related lymphomas, malignant immunoproliferative diseases, multiple myeloma and malignant plasma cell neoplasms, lymphoid leukemia, myeloid leukemia, acute or chronic lymphocytic leukemia, monocyte leukemia, other specific cell type leukemias, non-specific cell type leukemias, other and unspecified malignant lymphoid neoplasms, hematopoietic and related tissues, such as large cell lymphoma, T-cell lymphoma or cutaneous T-cell lymphoma). GPR182 was found to also be expressed in endothelial cells of the lymph nodes in humans (see, for example, Schmid CD etal., Biochem Biophys Res Commun. (2018);497(1):32-38). An inhibition of the GPR182 function (e.g., by GPR182 blockade) will hence also be helpful for the treatment of lymphatic cancers, e.g., by enhancing the anti-cancer immunity, enhancing the recruitment of immune cells (e.g., effector T cells) into affected lymph nodes and/or for immunotherapy against lymph nodes metastasis or lymphoma.

There may be other cancers which have not yet been investigated with respect to the presence or absence of GPR182 expression. However, means and methods for assessing the presence or absence of the expression of one or more cell surface proteins (such as GPR182) are well known and established in the art. The skilled person will hence be able to identify further cancers which are likely to benefit from the herein disclosed medical applications. For example, a tumor sample (e.g., obtained from biopsy) may be analyzed for GPR182 expression by immunofluorescent staining/imaging using anti-GPR182 antibodies which may be detected by means of a conjugated fluorescence label.

The nucleotide sequences of the GPR182-encoding gene and mRNA as well as the corresponding protein sequences are known for a number of species, including human GPR182, mouse GPR182, rat GPR182, and are available from the NCBI Database: https://www.ncbi.nlm.nih.gov). For example, the nucleotide sequence of the human GPR182 mRNA is available from the NCBI database (NCBI Accession ID: NM_007264.4; https://www.ncbi.nlm.nih.gov/nuccore/NM_007264) and defined herein by **SEQ ID NO: 1**; and the amino acid sequence of the full-length human GPR182 protein is available from the NCBI database (NCBI Accession ID.: AAH34761.1; https://www.ncbi.nlm.nih.gov/protein/AAH34761.1) and defined herein by **SEQ ID NO: 2**. Furthermore, also natural genetic variations of GPR182 have been described, including common genetic polymorphisms. One natural variant of GPR182 comprises a single amino acid change from cysteine to arginine at position 349 of the human GPR182 protein, which has a frequency of more than 10% in the general population. In view of Cys349 not being conserved in GPR182 among mammals (e.g., absent in murine GPR182 (UniProt Database entry: G3X9R9), it is reasonable to expect that this variation does not affect the function of the GPR182. The amino acid sequence of this natural variant (Cys349Arg) of human GPR182 is defined herein by **SEQ ID NO: 3**. It is hence understood that the term "GPR182" as referred to herein also embraces naturally occurring variants of GPR182, such as defined by SEQ ID NO: 3.

The term "naturally occurring variant" as used herein refers to variants of the GPR182 protein or GPR182-encoding nucleotide sequence which exist naturally within the defined taxonomic group, such as mammalian, such as mouse, monkey, and preferably human. Typically, when referring to "naturally occurring variants" of a GPR182-encoding polynucleotide, the term may also encompass any allelic variant of the GPR182-encoding genomic DNA that is found in the genome by chromosomal translocation or duplication, and/or the RNA, such as mRNA derived therefrom. For example, naturally occurring variants of the human GPR182 protein include the Cys349Arg variant as defined by SEQ ID NO: 3. Naturally occurring variants may also include variants derived from alternative splicing of the GPR182 mRNA. When referenced to a specific protein sequence, the term may also include naturally occurring forms of the protein which are processed, for example, by co- or post-translational modifications (e.g., proteolytic cleavage, glycosylation, etc.).

The term "endogenous ligand" as used herein means any molecule that is endogenously found in the subject (e.g., a certain mammal) envisaged for the herein disclosed medical/therapeutic applications and which is capable of interacting (*in vivo*) with the endogenously expressed GPR182 protein. The skilled person understands that a potential capacity of a ligand to bind to GPR182 protein can be assessed by *in vitro* experiments whereby a detected binding may also be indicative of a binding *in vivo.* Generally, such ligands include intracellular and extracellular ligands, and may be (poly)peptides, nucleic acids, lipids, sugars (incl. glycans and glycosphingolipids), metal ions, other naturally occurring molecules, and/or post-translational modifications.

In preferred embodiments, the endogenous ligand comprises or consists of a (poly)peptide (i.e., a (poly)peptide endogenously expressed in the envisaged subject), more preferably an endogenous chemokine ligand.

As used herein, "chemokines" or "chemotactic cytokines", are a family of small cytokines or signaling proteins secreted by cells that induce directional movement of leukocytes, as well as other cell types, including endothelial and epithelial cells. In addition to playing a major role in the activation of host immune responses, chemokines are important for biological processes, including morphogenesis and wound healing, as well as in the pathogenesis of diseases like cancers. Cytokine proteins are classified as chemokines according to behavior and structural characteristics. In addition to being known for mediating chemotaxis, chemokines are all approximately 8-10 kilodaltons in mass and have four cysteine residues in conserved locations that are key to forming their 3-dimensional shape. These proteins have historically been known under several other names including the SIS family of cytokines, SIG family of cytokines, SCY family of cytokines, Platelet factor-4 superfamily or intercrines. Some chemokines are considered pro-inflammatory and can be induced during an immune response to recruit cells of the immune system to a site of infection, while others are considered homeostatic and are involved in controlling the migration of cells during normal processes of tissue maintenance or development. Chemokines are found in all vertebrates, some viruses and some bacteria, but none have been found in other invertebrates. Chemokines have been classified (based on the position of one or more cysteines in their N-terminus) into four main subfamilies: CXC, CC, CX3C and C. All of these proteins exert their biological effects by interacting with G protein-linked transmembrane receptors (i.e., G-protein coupled receptors (GPCRs)) called chemokine receptors, that are selectively found on the surfaces of their target cells. Chemokines are functionally divided into two groups: homeostatic and inflammatory. Homeostatic chemokines are constitutively produced in certain tissues and are responsible for basal leukocyte migration. These include: CCL14, CCL19, CCL20, CCL21, CCL25, CCL27, CXCL12 and CXCL13. This classification is not strict; for example, CCL20 can act also as pro-inflammatory chemokine. Inflammatory chemokines are formed under pathological conditions (on pro-inflammatory stimuli, such as IL-1, TNF-alpha, LPS, or viruses) and actively participate in the inflammatory response attracting immune cells to the site of inflammation. Examples are: CXCL8, CCL2, CCL3, CCL4, CCL5, CCL11, CXCL10.

The term "nucleotide sequence", as used herein interchangeably with the terms "nucleic acid sequence" or "polynucleotide sequence", refers to polynucleotides including deoxyribonucleic acid (DNA), such as cDNA or genomic DNA, and, where appropriate, ribonucleic acid (RNA). It is understood that the term "RNA" as used herein comprises all forms of RNA including mRNA.

The term "protein", as interchangeably used herein with the term "polypeptide", refers to a linear polymer of amino acid residues linked by peptide bonds in a specific sequence. The group of "polypeptides" consists of molecules with more than 30 amino acids, which is in distinction to the group of "peptides" which consists of molecules with up to 30 amino acids. The group of "peptides" also refers to fragments of proteins of a length of 30 amino acids or less. As used herein, the term "(poly)peptides" refers more generally to both "peptides" and "polypeptides". (Poly)peptides may further form dimers, trimers and higher oligomers, i.e., consisting of more than one (poly)peptide molecule. (Poly)peptide molecules forming such dimers, trimers etc. may be identical or non-identical. The corresponding higher order structures are, consequently, termed homo- or heterodimers, homo- or heterotrimers etc. Homo- or heterodimers etc. also fall under the definition of the term "(poly)peptide". The term "(poly)peptide" also refers to chemically or co-/post-translationally modified peptides and polypeptides.

The term "specifically binds" or "binds specifically", as interchangeably used throughout the present specification in relation to the agent (e.g., an antibody) of the invention, means that the agent binds the GPR182 protein, while preferentially having no or only insignificant binding affinity to other (poly)peptides and/or other molecules present in the subject (i.e., the subject for which the therapeutic application is envisaged); for example, in particular, to those other (poly)peptide(s) or other molecule(s) which upon interaction with the agent may exert any undesired effect(s), such as inducing a signalling that would be detrimental to the envisaged effects and therapeutic applications contemplated herein and/or have any otherwise negative impact on the subject's health which would outweigh an envisaged therapeutic benefit. The term, however, does not exclude the fact that an agent of the invention may also be cross-reactive with a GPR182 homolog, ortholog or paralog from another mammalian species. That an agent "specifically binds" to the GPR182 protein is preferentially understood to mean that the binding affinity between the agent and the GPR182 protein is at least (with increasing preference) 1.5-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, 20-fold, 21-fold, 22-fold, 23-fold, 24-fold, 25-fold, 26-fold, 27-fold, 28-fold, 29-fold, 30-fold, 31-fold, 32-fold, 33-fold, 34-fold, 35-fold, 36-fold, 37-fold, 38-fold, 39-fold, 40-fold, 45-fold, 50-fold, 60-fold, 70-fold, 80-fold, 90-fold, 100-fold, 200-fold, 300-fold, 400-fold or 500-fold or even higher as compared to the binding affinity between the agent and other (poly)peptide(s) and/or other molecule(s) present in the subject. Preferably, the other (poly)peptide(s) as referred to in this latter embodiment is (are) selected from one or more of the C-X-C chemokine receptor type 4 (CXCR4) and atypical chemokine receptor 3 (ACKR3, also known as CXCR7). Even more preferably, the agent does not bind to CXCR4 and ACKR3 (CXCR7).

In any case, the skilled person will be able to assess an agent (i.e., a candidate agent) for its capacity to specifically bind to the GPR182 protein and to assess a potential binding of the agent to other (poly)peptides/molecules using conventional (e.g., biophysical) methods, such as, without limitation, isothermal titration calorimetry (ITC), surface plasmon resonance, and/or (competitive) binding assays as known and routinely employed in the art, and as also described herein (see, e.g., Example 2).

The term "inhibits the binding" (*i*.*e*., of the one or more endogenous ligands to the GPR182 protein) in the context of the agent of the present invention refers to the ability of said agent, based on its capacity to specifically bind to the GPR182 protein, to prevent (e.g., to block) the binding of the one or more endogenous ligands (*i*.*e*., ligand(s) of the GPR182 protein). For example, an agent may specifically bind to the GPR182 protein at, or in proximity to, the binding site(s) of the one or more endogenous ligands and thereby render the binding site on the GPR182 protein inaccessible or thermodynamically/energetically less favorable, e.g., by providing a steric hindrance and/or an electrostatic repulsion, for the binding of the one or more endogenous ligands to the GPR182 protein. An agent may hence compete with the one or more endogenous ligands for the binding to the GPR182 protein. Alternatively, the agent may bind to an allosteric site of the GPR182 protein, which allosteric may be a site that is distinct and distant to a binding site of the one or more endogenous ligands on the GPR182 protein, and wherein a binding of the agent to the GPR182 protein induces a conformational change of the GPR182 protein, which leads to a distortion of the binding site(s) of the one or more endogenous ligands, in consequence of which the one or more endogenous ligands are unable (or at least negatively affected) to bind the correspondingly distorted GPR182 protein.

Whereas it is generally understood by the skilled artisan in view of the purposes contemplated herein that such agents are preferred which do not itself bind (*i*.*e*., no binding affinity) to the one or more endogenous ligands, it is in any case to be understood that the agent, if also having any binding affinity towards the one or more endogenous ligands, has a higher binding affinity towards the GPR182 protein as compared to its binding affinity to the one or more endogenous ligands, wherein the "higher binding affinity" is preferably understood to mean a binding affinity that is at least (with increasing preference) 1.5-fold, 2-fold, 3-fold, 4-fold, 5-fold, 6-fold, 7-fold, 8-fold, 9-fold, 10-fold, 11-fold, 12-fold, 13-fold, 14-fold, 15-fold, 16-fold, 17-fold, 18-fold, 19-fold, 20-fold, 21-fold, 22-fold, 23-fold, 24-fold, 25-fold, 26-fold, 27-fold, 28-fold, 29-fold, 30-fold, 31-fold, 32-fold, 33-fold, 34-fold, 35-fold, 36-fold, 37-fold, 38-fold, 39-fold, 40-fold, 45-fold, 50-fold, 60-fold, 70-fold, 80-fold, 90-fold, 100-fold, 200-fold, 300-fold, 400-fold or 500-fold or even higher as compared to the binding affinity between the agent and the one or more endogenous ligand(s).

Moreover, generally such agents are preferred which non-covalently (i.e., reversibly) bind to the GPR182 protein. However, alternatively, also such agents are contemplated herein which under physiological conditions may be capable of undergoing a covalent (i.e., an irreversible) interaction with the GPR182 protein.

It is understood that the ability of the agent to inhibit (or prevent or block) the binding of one or more endogenous ligands means that the binding of the latter to the GPR182 protein is at least partially inhibited (i.e., prevented and/or blocked), preferably, with increasing preference, by at least (with increasing preference) 30%, 40%, 50%, 60%, 70%, 80%, 90%, 91%, 92%, 93%, 94%, 95%, 96%, 97%, 98%, 99% or more. Whereas the skilled person understands that an (at least partial) inhibition of the binding of the one or more endogenous ligands to the GPR182 protein may also be achieved by agents having a weaker binding affinity to the GPR182 protein as compared to the binding affinity between the one or more endogenous agents to the GPR182 protein, *i*.*e*., by adjusting the dose/concentration of the agent to shift the binding equilibrium towards the complex of the agent and the GPR182 protein, it is generally preferred that the agent has a higher binding affinity (*i*.*e*., a lower dissociation constant (K_{D})) to the GPR182 protein as compared to the binding affinity between the one or more endogenous ligands and the GPR182 protein. The strength of an inhibition is typically measured by assessing the concentration of half-maximal inhibition (IC₅₀). The term inhibition and the assessment of IC₅₀ values are well established in the field.

In accordance with the invention, the "agent" that specifically binds to the GPR182 protein is selected from an antibody, an Fc fusion polypeptide, an adnectin, an affibody, an affilin, an anticalin, an atrimer, an avimer, a designed ankyrin repeat protein (DARPin), an evibody, a fynomer, a Kunitz-type domain, a peptide or peptidomimetic, an aptamer and a small molecule.

The term "antibody", as used herein, comprises, for example, polyclonal or monoclonal antibodies. Furthermore, also derivatives or fragments thereof, which still retain the binding specificity to the target, in the present case the GPR182 protein or a particular portion thereof, are encompassed by the term "antibody". Antibody fragments or derivatives comprise, *inter alia*, Fab or Fab' fragments, Fd, F(ab')₂, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigens. For example, the first antigen can be found on the GPR182 protein, and the second antigen may, for example, be a tumor marker that is specifically expressed on cancer cells or a certain type of cancer cells. Non-limiting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847).

The term "antibody", as used herein, also refers to chimeric (human constant domain, non-human variable domain), single chain and humanized (human antibody with the exception of non-human CDRs) antibodies.

Various techniques for the production of antibodies are well known in the art and described, e.g., in Harlow and Lane (1988) and (1999) and Altshuler EP, et al., Biochemistry (Mosc). 2010 Dec;75(13):1584-605. Thus, polyclonal antibodies can be obtained from the blood of an animal following immunization with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Kohler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared *de novo* using various display methods such as phage, ribosomal, mRNA, or cell display (see, e.g., Hoogenboom HR. Selecting and screening recombinant antibody libraries. Nat Biotechnol. 2005 Sep;23(9):1105-16). A suitable system for the expression of the recombinant (humanized) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope on the GPR182 protein.

The term "antibody", as used herein, also refers to "nanobodies (Nbs)". The term "nanobody", also known as a "single-domain antibody (sdAb)", is an antibody fragment consisting of a single monomeric variable antibody domain. The first single-domain antibodies were engineered from heavy-chain antibodies found in camelids; these are called VHH fragments. Cartilaginous fishes also have heavy-chain antibodies (IgNAR, 'immunoglobulin new antigen receptor'), from which single-domain antibodies called VNAR fragments can be obtained. An alternative approach is to split the dimeric variable domains from common immunoglobulin G (IgG) from humans or mice into monomers. Although most research into single-domain antibodies is currently based on heavy chain variable domains, nanobodies derived from light chains have also been shown to bind specifically to target epitopes. Nanobodies can be efficiently selected from large (semi-) synthetic/naive or immunized cDNA-libraries using well established display technologies like phage- or yeast-display. The simple and single-gene format enables the production of purified nanobodies in the mg-g range per liter of culture, thereby offering an unlimited supply of consistent binding molecules. Additionally, nanobodies can be easily genetically or chemically engineered. Nanobodies are characterized by high affinities and specificities, robust structures, including stable and soluble behaviors in hydrophilic environments and superior cryptic cleft accessibility, low-off target accumulation, and deep tissue penetration. To date, many nanobodies have been evolved into versatile research and diagnostic tools and the list of therapeutic nanobodies applied in clinical trials is constantly growing. Nanobody-derived formats comprise the nanobody itself, homo- or heteromultimers, nanobody-coated nanoparticles or matrixes, nanobody-displayed bacteriophages or enzymatic-, fluorescent- or radionuclide-labeled nanobodies. All these formats were successfully applied in basic biomedical research, cellular and molecular imaging, diagnosis or targeted drug delivery and therapy (see, for example, Chames P, Rothbauer U. Special Issue: Nanobody. Antibodies (Basel). 2020 Mar 6;9(1):6). Methods for the generation and target-specific selection of nanobodies are well-established in the art; see for example, Salema V et al., Escherichia coli surface display for the selection of nanobodies. Microb Biotechnol. 2017 Nov;10(6):1468-1484). Embodiments wherein the agent that specifically binds to the GPR182 protein is a "nanobody" are particularly preferred.

In accordance with the invention, an "agent" can be an "Fc-fusion polypeptide". The latter term refers to a fusion construct of a binding domain (e.g., a single-chain antibody or an antigen binding protein of any alternative scaffold, e.g., as referred to herein) with an antibody Fc-region.

Moreover, the term "agent", as used herein, also embraces antibody mimetics, i.e., antigen binding molecules which, like antibodies, can specifically bind antigens, such as the GPR182 protein in the present case, but which are not structurally related to antibodies, *i*.*e*., are derived from alternative structural scaffolds. A multitude of such alternative (e.g., non-antibody) binding protein scaffolds has been developed in the last decades (as reviewed for example, in Hosse RJ et al. Protein Sci. 2006;15(1):14-27; Weidle UH, et al., (2013), Cancer Genomics & Proteomics; 10(4):155-68) and it is contemplated that any of these may be suitably employed as an agent for the purposes of the present invention.

Exemplary antibody mimetics which are particularly contemplated herein, yet without intended to be limiting, are adnectins, affibodies, affilins, anticalins, atrimers, avimers, designed ankyrin repeat proteins (DARPins), evibodies, fynomers, and Kunitz-type domains.

"Adnectins", as used herein, refer to a class of binding proteins having a scaffold which consists of a backbone of the natural amino acid sequence of the 10^{th} domain of the 15 repeating units of human fibronectin type III (FN3). The molecule adopts a β-sandwich fold with seven strands connected by six loops similar like an immunoglobulin domain, but without any disulfide bonds. Three loops at one end of the β-sandwich can be engineered to enable an adnectin to specifically recognize a therapeutic target of interest. Non-loop residues have also been found to expand the available binding footprint. Ligand-binding adnectin variants with binding affinities in the nanomolar to picomolar range have been selected via mRNA, phage, and yeast display (Hackel BJ, et al. (2008) J Mol Biol 381:1238-1252).

"Affibodies", as used herein, refer to a class of binding proteins that is derived from the Z-domain of staphylococcal protein A. Affibodies are structurally based on a three-helix bundle domain. An affibody has a molecular mass of around 6 kDa and is stable at high temperatures and under acidic or alkaline conditions. Target specificity is obtained by randomization of amino acids located in two alpha-helices involved in the binding activity of the parent protein domain (Feldwisch, J & Tolmachev, V. (2012) Methods Mol. Biol. 899:103-126). Methods of making affibodies are known in the art and are described in Wikman M et al., Protein Eng Des Sel. (2004);17(5):455-62.

"Affilins", as used herein, refer to a class of binding proteins that are developed by using either gamma-B crystalline or ubiquitin as a scaffold and modifying amino-acids on the surface of these proteins by random mutagenesis. Selection of affilins with the desired target specificity, *i*.*e*., against GPR182, is effected, for example, by phage display or ribosome display techniques. Depending on the scaffold, affilins have a molecular weight of approximately 10 or 20 kDa. As used herein, the term affilin also refers to di- or multimeric forms of affilins (Weidle UH, et al., (2013), Cancer Genomics & Proteomics; 10(4):155-68).

"Anticalins", as used herein, refer to a class of engineered proteins derived from a lipocalin. Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape (see, e.g., Beste G, Schmidt FS, Stibora T, Skerra A. (1999) Proc Natl Acad Sci USA. 96(5):1898-903; Gebauer and Skerra (2009) Curr Opinion in Chemical Biology 13:245-255); Rothe C & Skerra A., BioDrugs. (2018);32(3):233-243; Gebauer M & Skerra A, Curr Opin Biotechnol. (2019); 60:230-241).

"Atrimers", as used herein, refer to binding molecules which underlying scaffold is derived from the protein tetranectin. Tetranectin is a member of the C-type lectin family which is characterized by the C-type lectin domain (CTLD). It is composed of three identical chains possessing a C-terminal trimerizing coil-coil region. The formation of the homotrimer leads to an apparent 100-fold increase in affinity for its cognate ligand, which is probably due to an avidity effect based on the three-fold clustering of the CTLDs. Each CTLD has five loop regions, 6-9 amino acids in length, which mediate binding specificity. The sequence of these loops can be changed without perturbing the overall structure. Accordingly, monomeric CTLDs can be displayed on phage libraries for the selection of binders to specific targets, but trimeric versions were used for later applications. Atrimers with molecular weights ranging between 60-70 kDa are much smaller than antibodies, are not glycosylated and might be endowed with better tissue penetration in comparison to antibodies (Weidle UH, et al., (2013), Cancer Genomics & Proteomics; 10(4):155-68)

"Avimers" (short for avidity multimers), as used herein, are a class of artificial multi-domain proteins which specifically bind certain antigens via multiple binding sites. This protein is also known as "maxibody" or low-density lipoprotein receptor (LDLR) domain A. It consists of two or more (poly)peptide sequences, which are based on A domains. The A domains are 30 to 35 amino acids scaffolds (~4 kDa) derived from extracellular cysteine-rich cell surface receptor proteins and stabilized by disulfide bond formation and complexation of calcium ions. The scaffold structure is maintained by 12 conserved amino acids, leaving all the remaining non-conserved residues amenable to randomization and ligand binding. Avimers are highly thermostable. Due to their small size, avimers often consist of multiple A-domains with each binding to a different site on the target, thereby achieving increased affinity through avidity (Silverman J et al. (2005), Nat Biotechnol 23:1556-1561).

"DARPins", as used herein, are designed ankyrin repeat domains and based on tightly packed ankyrin repeats, each forming a β-turn and two antiparallel α-helices. DARPins usually carry three repeats corresponding to an artificial consensus sequence, whereby a single repeat typically consists of 33 amino acids, six of which form the binding surface. During recombinant library design, these sites are used to introduce the codons of random amino acids. DARPins are typically formed by two or three of the binding motifs contained between the N- and C-terminal motifs shielding the hydrophobic regions. DARPins are small proteins (~14-18 kDa) that are extremely thermostable and resistant to proteases and denaturing agents (Plückthun A., Annu Rev Pharmacol Toxicol. (2015); 55:489-511).

"Evibodies", as used herein, are engineered binding proteins derived from the variable(V)-set Ig-like scaffold of the T-cell surface receptor Cytotoxic T Lymphocyte-associated Antigen 4 (CTLA-4). Loops corresponding to CDRs of antibodies can be substituted with heterologous sequences to confer different binding properties. Methods of making Evibodies are known in the art and described, for example, in U.S. Patent No. 7,166,697.

The term "fynomer" (or "Fynomer^{®}"), as used herein refers to a non-immunoglobulin-derived binding polypeptide derived from the human Fyn SH3 domain. Fyn SH3-derived polypeptides are well-known in the art and have been described, e.g., in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "Kunitz-type domain" or "Kunitz domain-based binder", refers to a class of binding proteins derived from the active motif of Kunitz-type protease inhibitors. The latter proteins comprise loop regions that can be mutated without destabilizing the structural framework. The hydrophobic core of a Kunitz domain is composed of a twisted two-stranded antiparallel β-sheet and two α-helices stabilized by three pairs of disulfide bonds (Hosse RJ et al. (2006). Protein Sci 15:14-27; Simeon R. & Chen Z. Protein Cell. (2018);9(1):3-14).

Means and methods for developing, screening and identification of suitable antigen binding molecules (e.g., (poly)peptides of various scaffolds, including, without being limiting, those described herein above) towards desired target structures (such as the GPR182 protein or certain portions thereof) are well known and routinely employed in the art. Exemplary nowadays routinely-performed methods include, without intended to being limiting, high-throughput (HT) combinatorial library-based display and selection methods, such as phage display, ribosome display, mRNA display, and cell surface display (e.g. yeast display); see, for example, Davydova, et al. Biochemistry Moscow 87, S146-S167 (2022); Frei JC, Lai JR. Protein and Antibody Engineering by Phage Display. Methods Enzymol. 2016;580:45-87;.

In accordance with the invention, the "agent" that specifically binds to the GPR182 protein may also be a "peptidomimetic".

The term "peptidomimetic", as used herein, refers to a molecule containing non-peptidic structural elements that is capable of mimicking or antagonizing the biological action(s) of a natural peptide. Thus, the term "peptidomimetic", as used herein, may referto any sequence that is designed to mimic a peptide structure and/or function, but in which the backbone is not based on α-amino acid residues alone. To date, numerous strategies have been explored - from incorporation of a single unnatural amino acid residue to alternative backbone structures - in orderto discover potent peptidomimetic-based drug leads exhibiting high cell selectivity and improved bioavailability. Peptidomimetics may for example comprise N-alkylated glycines (peptoids), β-peptoids (N-alkylated β-alanine oligomers), β-peptides, α-peptide/β-peptoids, α/γ N-acylated N-aminoethylpeptides (AApeptides) and oligoacyllysines (OAKs). An advantage of such peptidomimetics is that solid-phase synthesis allows for convenient preparation of large arrays of analogs by simple replacement/insertion of alternative residues by way of commercial and/or synthetic amino acid analogs. Also, these peptidomimetics often possess enhanced *in vivo* stability (see, e.g., Vagner J, Qu H, Hruby VJ. Peptidomimetics, a synthetic tool of drug discovery. Curr Opin Chem Biol. 2008;12(3):292-296).

In accordance with the invention, the "agent" that specifically binds to the GPR182 protein may also be an "aptamer".

"Aptamers" are nucleic acid molecules or peptide molecules that are capable of binding to a specific target with high affinity and specificity. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

Nucleic acid aptamers are nucleic acid species that normally consist of (usually short) strands of oligonucleotides (e.g., DNA or RNA of 15-100 nt length). Aptamers to almost any molecular target (such as small molecules, proteins, nucleic acids, and even cells, tissues and organisms) can be obtained using an *in vitro* evolutionary approach called SELEX (systematic evolution of ligands by exponential enrichment) (see, e.g., Ellington A.D., Szostak J.W. Nature. 1990; 346:818-822; Santosh B, Yadava PK. Nucleic acid aptamers: research tools in disease diagnostics and therapeutics. Biomed Res Int. 2014;2014:540451). SOMAmers (slow off-rate modified aptamers) are DNA aptamers in which nucleic bases are modified with some hydrophobic moieties. These modifications expand the range of aptamer-target interactions with hydrophobic ones, which are absent in conventional DNA and RNA aptamers. This, in turn, results in higher affinities of SOMAmers to their targets (see, e.g., Elskens et. al., Int. J. Mol. Sci. 2020; 21:4522).

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A (TrxA) is the most commonly used scaffold protein. TrxA has an active site composed of a Cys-Gly-Pro-Cys stretch which is capable of accommodating long peptide insertions albeit with a concomitant loss of enzymatic function. Peptide aptamer selection can be made using different systems, but the most widely used is currently the yeast two-hybrid system (see Reverdatto S, et al. Peptide aptamers: development and applications. Curr Top Med Chem. 2015;15(12):1082-101).

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half-life extending proteins etc. are available to scientists such that the half-life of aptamers can be increased for several days or even weeks.

The term "small molecule", as used herein, is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g., N, S, or O. For all above-described organic molecules the molecular weight is preferably in the range of 200 Da to 1500 Da and more preferably in the range of 300 Da to 1000 Da. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about 250 Da. The size of a small molecule can be determined by methods well-known in the art, e.g., by mass spectrometry. Small molecules may be rationally designed, for example, based on structural information available from predicated structural models (see for example, https://alphafold.ebi.ac.uk/entry/O15218 for a structural model of human GPR182) or experimentally determined high-resolution structures (e.g., by X-ray crystallography, NMR spectroscopy or Cryo-EM) of the target molecule, where sites presumably responsible for the biological activity, such as a ligand binding site on a receptor, can be identified and verified, *e*.*g*., in *in vivo* assays such as *in vivo* high-throughput screening (HTS) assays.

In accordance with the invention, the "agent" that inhibits the expression of the GPR182 protein is selected from an siRNA, miRNA, shRNA, a ribozyme and/or an antisense nucleic acid molecule.

As used herein, the term "small interfering RNA (siRNA)", also known as "short interfering RNA" or "silencing RNA", refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways e.g., as an antiviral mechanism or in shaping the chromatin structure of a genome. siRNAs naturally found in nature have a well-defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene for which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1 to 5 nucleotides, more preferably from 1 to 3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. Nature. 2001;411(6836):494-8). 2'-deoxynucleotides in the 3' overhangs are as efficient as ribonucleotides but are often cheaper to synthesize and probably more nuclease resistant. Methods for designing and preparing siRNAs (including siRNA design software) suitable for effectively knocking down the expression of selected target genes are established and can be routinely employed by the skilled person (see, e.g., Naito Y, et al. Front Genet. 2012; and the review article by Fakhr E et al., Cancer Gene Ther. 2016;23(4):73-82). In addition, various companies (e.g., ThermoFisher, https://www.thermofisher.com) offer online design tools for siRNAs as well as manufacturing services. Delivery of siRNA may be accomplished using any of the methods known in the art. For example, two of the most popular methods of delivering siRNA therapies are liposome-based systems and nanoparticle-based systems. SiRNAs may be combined with saline and the combination can be administered intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics; Tatiparti K et. al. siRNA Delivery Strategies: A Comprehensive Review of Recent Developments. Nanomaterials (Basel). 2017;7(4):77; Paunovska, K. et al., Drug delivery systems for RNA therapeutics. Nat Rev Genet 23, 265-280 (2022).).

A "short hairpin RNA (shRNA)" is a sequence of RNA that makes a tight hairpin turn that can be used to silence gene expression via RNA interference. shRNA typically uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer.

Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, "microRNAs (miRNAs)". Said RNA species are single-stranded RNA molecules. Endogenously present miRNA molecules regulate gene expression by binding to a complementary mRNA transcript and triggering of the degradation of said mRNA transcript through a process similar to RNA interference. Accordingly, exogenous miRNA may be employed as an inhibitor of GPR182 expression after introduction into the respective cells.

A "ribozyme" (from ribonucleic acid enzyme, also called RNA enzyme or catalytic RNA) is an RNA molecule that catalyses a chemical reaction. Many natural ribozymes catalyse either their own cleavage or the cleavage of other RNAs, but they have also been found to catalyse the aminotransferase activity of the ribosome. Non-limiting examples of well-characterised small self-cleaving RNAs are the hammerhead, hairpin, hepatitis delta virus, and in vitro-selected lead-dependent ribozymes, whereas the group I intron is an example for larger ribozymes. The principle of catalytic self-cleavage has become well established in recent years. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: A region of interest of the RNA, which contains the GUC (or CUC) triplet, is selected. Two oligonucleotide strands, each usually with 6 to 8 nucleotides, are taken and the catalytic hammerhead sequence is inserted between them. The best results are usually obtained with short ribozymes and target sequences.

A more recent development, also useful in accordance with the present invention, is the combination of an aptamer, recognizing a small compound, with a hammerhead ribozyme. The conformational change induced in the aptamer upon binding the target molecule can regulate the catalytic function of the ribozyme.

The term "antisense oligonucleotide (ASO)", as used herein, refers to single-stranded DNA or RNA molecules that are complementary to a chosen target nucleic acid. An antisense nucleic acid molecule, in accordance with the invention, is capable of interacting with the target nucleic acid, more specifically it is capable of hybridizing with the target nucleic acid. Due to the formation of the hybrid, transcription of the target gene(s) and/or translation of the target mRNA is reduced or blocked. For example, an antisense RNA is a single-stranded RNA molecule that is complementary to a protein coding mRNA (mRNA) with which it hybridizes, and thereby blocks its translation into protein. Standard methods relating to antisense technology have been described (see, e.g., Melani et al., Cancer Res. (1991) 51 :2897-2901).

Moreover, in alternative preferred embodiments, the "agent" that inhibits the expression of the GPR182 protein may be a nucleotide-based inhibitor, wherein the nucleotide-based inhibitor comprises or consists of:
(a) a polynucleotide sequence which comprises or consists of a polynucleotide sequence being complementary to at least 12 continuous nucleotides of a nucleic acid sequence encoding GPR182 protein (preferably SEQ ID NO: 1);
(b) a polynucleotide sequence which comprises or consists of a nucleic acid sequence which is at least 70% identical to the complementary strand of one or more nucleic acid sequences encoding GPR182 protein (preferably SEQ ID NO: 1);
(c) a polynucleotide sequence which comprises or consists of a polynucleotide sequence according to (a) or (b), wherein the polynucleotide sequence is DNA or RNA;
(d) an expression vector expressing the polynucleotide sequence as defined in any one of (a) to (c), preferably under the control of a tissue-specific promoter (in cases where the targeted medical condition is a cardiac condition (e.g., MI), preferably a heart-tissue-specific promoter); or
(e) a host comprising the expression vector of (d).

A "promoter" is a nucleic acid sequence that initiates transcription of a particular gene or a polynucleotide (e.g., a nucleotide-based inhibitor as referred to in the above embodiment). The promoter can be a constitutively active promoter, a tissue-specific or development-stage-specific promoter, an inducible promoter, or a synthetic promoter. Constitutive promoters direct expression in virtually all tissues and are largely, if not entirely, independent of environmental and developmental factors. As their expression is normally not conditioned by endogenous factors, constitutive promoters are usually active across species and even across kingdoms. Tissue-specific or development-stage-specific promoters direct the expression of a gene in specific tissue(s) or at certain stages of development. The activity of inducible promoters is induced by the presence or absence of biotic or abiotic factors. Inducible promoters are a very powerful tool in genetic engineering because the expression of genes operably linked to them can be turned on or off as needed. Synthetic promoters are constructed by bringing together the primary elements of a promoter region from diverse origins. In embodiments where the targeted medical condition is a cardiac disease (e.g., MI), the expression vector preferably contains a heart-specific promoter. Heart-specific promoters are known in the art, for example, from Boecker et al. (2004), Mol Imagin.; 3(2):69-75. Using a heart-specific promoter ensures that the nucleic acid sequence is only expressed in the heart and may avoid potential unwanted side effects by expression in other organs.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 70%, preferably at least 80%, more preferably at least 90%, and most preferred at least 95% are envisaged by the invention. However, also envisaged by the invention are with increasing preference sequence identities of at least 97.5%, at least 98.5%, at least 99%, at least 99.5%, at least 99.8%, and 100%.

It will be appreciated that the herein disclosed medical uses and applications are useful in the fields of human medicine and veterinary medicine. Thus, the term "subject" or "patient" as used herein refers to any vertebrate including, without limitation, humans and other primates (e.g., chimpanzees and other apes and monkey species), farm animals (e.g., cattle, sheep, pigs, goats and horses), domestic mammals (e.g., dogs and cats), laboratory animals (e.g., rodents such as mice, rats, rabbits, guinea pigs and hamsters), and birds (e.g., domestic, wild and game birds such as chickens, turkeys and other gallinaceous birds, ducks, geese, and the like). In preferred embodiments, the subject is a mammal. In even more preferred embodiments, the subject is a human. Whereas it is preferentially understood that the term "subject" refers to a mammal which endogenously expresses a GPR182, also such mammals are intended to be embraced by the term subject which heterologously express a GPR182 gene/protein from another mammal, for example, a mouse expressing the human GPR182 protein as used in the herein disclosed examples.

GPR182 has been found by the inventors to undergo constitutive internalization (endocytosis) in a ligand-independent manner and that this causes a local removal of its endogenous chemokine ligands from plasma at sites of GPR182 expression. GPR182 is hence thought to function as a scavenging receptor which acts in controlling the plasma levels and/or the tissue levels of its endogenous chemokine ligands. In view of the further findings that the absence of this GPR182 functionality causes an increase of the plasma availability of its endogenous chemokine ligands (esp. CXCL12) and the observed advantageous effects caused thereby, it is particularly contemplated herein that the inhibition of the binding of the one or more endogenous ligands affected by the agent prevents the GPR182-mediated internalization of the one or more endogenous ligands. Thus, in a preferred embodiment, the binding of the agent to the GPR182 protein inhibits the GPR182-mediated internalization of the one or more endogenous GPR182-ligands. Moreover, considering the possibility that the GPR182 protein after internalization may cycle back to the cell surface in consequence of which its scavenging capacity may be restored, it is even more preferred that the binding of the agent to the GPR182 protein prevents the GPR182 from internalization. Means and methods for assessing a potential inhibition of a receptor-mediated ligand internalization are known in the art and are also described, e.g., in Le Mercier *et al.* 2021.

Yet, in alternative preferred embodiments, the agent, upon binding to the GPR182 protein, promotes GPR182 protein internalization and lysosome-dependent degradation by directing the GPR182 protein to the endosomal-lysosomal degradation pathway. In further alternative preferred embodiments, the agent, upon binding to the GPR182 protein, promotes GPR182 protein internalization and proteasome-dependent degradation by directing the GPR182 protein into the ubiquitin-proteasome system (UPS). The feasibility of such an approach for inducing the targeted protein degradation of a GPCR was demonstrated in a recent study by Li *et al.* using Proteolysis targeting chimeras (PROTACs) demonstrated (in that case α1A-adrenergic receptor); see, e.g., Li, Z. Z. et al., Acta Pharm. Sin. B. 10, 1669-1679 (2020); and review by He M et al., Signal Transduct Target Ther. 2022 Jun 9;7(1):181.

As discussed herein above, GPR182 was found to act as a receptor for various CXC-type chemokines (cf. Le Mercier *et. al.*, 2021; Torphy *et al.,* 2022). Thus, in preferred embodiments, the one or more endogenous ligand(s) are one or more C-X-C chemokine ligand(s). For example, the one or more endogenous ligand(s) may be selected from C-X-C motif chemokine ligand 9 (CXCL9), CXCL10, CXCL11, CXCL12, and/or CXCL13.

In an even more preferred embodiment, the one or more endogenous ligands are selected from one or more of C-X-C motif chemokine ligand 10 (CXCL10), C-X-C motif chemokine ligand 12 (CXCL12) and/or C-X-C motif chemokine ligand 13 (CXCL13). In an even further preferred embodiment, the one or more endogenous ligands is (are) (i) CXCL12; or (ii) CXCL10 and CXCL12; or (iii) CXCL12 and CXCL13; or (iv) CXCL10 and CXCL13; or (v) CXCL10, CXCL12 and CXCL13. The latter two embodiments are particularly preferred in embodiments where the targeted pathological condition is myocardial infarction (MI) (or any post-complication(s) thereof), myocardial ischemia, myocardial necrosis, cardiac hypertrophy, cardiac fibrosis, limb ischemia, ischemia-related tissue degeneration and/or any another cardiovascular condition.

In other embodiments, the one or more endogenous ligands are selected from one or more of C-X-C motif CXCL9, CXCL10, and/or CXCL11. In a preferred embodiment of the latter embodiment, the one or more endogenous ligands is (are) (i) CXCL9; or (ii) CXCL10; or (iii) CXCL11; or (iv) CXCL9 and CXCL10; or (v) CXCL9 and CXCL11; or (vi) CXCL10 and CXCL11; or (vii) CXCL9, CXCL10 and CXCL11. The latter two embodiments are particularly preferred in embodiments where the targeted pathological condition is a cancer.

Structure prediction indicates that the GPR182 protein possesses a typical GPCR topology with an extracellular N-terminus, seven transmembrane helices and an intracellular C-terminus (see, e.g., for human GPR182, the corresponding prediction provided at the UniProt Database accession no.: 015218; https://www.uniprot.org/uniprot/O15218).

In preferred embodiments, the agent that specifically binds to the GPR182 protein specifically binds to one or more epitopes within one or more of the extracellular portions of the GPR182 protein.

According to structure predication, the extracellular portions of the human GPR182 protein consist of the amino acids 1-57, 114-127, 194-217, and 281-299 of the amino acid sequence defined by SEQ ID NO: 2 (see UniProt entry: 015218; https://www.uniprot.org/uniprot/O15218).

The term "epitope" or "binding site", as interchangeably used herein, relates to a portion, or portions, of a macromolecule, in the present case the GPR182 protein, which is capable of being bound by a specific binding member, i.e., an agent that specifically binds the GPR182 protein as referred to herein. Epitopes generally consist of chemically active surface groups and have specific three-dimensional structural characteristics, as well as specific charge characteristics. The epitope referred to herein is a "functional epitope" which is understood to mean that binding of the agent to the epitope has an effect on the function of the GPR182. In particular, binding of the epitope by an agent typically antagonises the biological activity of the GPR182 protein to interact with its one or more endogenous ligands. The epitope is therefore known as an "inhibiting" epitope or an "inhibitory" epitope. An "inhibiting" or "inhibitory" epitope means an epitope present on the GPR182 protein that when being bound by the agent results in loss or reduction of the biological activity of the GPR182.

Epitopes may be contiguous or non-contiguous sequences of amino acid residues comprised within a polypeptide sequence. The term "contiguous epitope" defines an epitope comprised of a linear series of amino acid residues within a polypeptide which define the epitope. A "non-contiguous epitope", which may also be referred to as a conformational or discontinuous epitope, is an epitope which is comprised of a series of amino acid residues which are non-linear in alignment, that is that the residues are spaced or grouped in a non-continuous manner along the length of a polypeptide sequence. A non-continuous epitope can be a discontinuous epitope wherein the amino acid residues are grouped into 2 linear sequences, or alternatively the non-continuous epitope can be a discontinuous scattered epitope wherein the residues which contribute to the epitope are provided in 3 or more groups of linear amino acid sequences arranged along the length of the polypeptide.

In particularly preferred embodiments, the one or more epitopes to which the agent specifically binds are comprised in:
(i) the N-terminal extracellular domain; and/or
(ii) the second extracellular loop (ECL2)
of the GPR182 protein.

As described herein above, according to the prediction provided at the UniProt Database (see UniProt accession no.: 015218; https://www.uniprot.org/uniprot/O15218), the N-terminal extracellular domain of human GPR182 (as defined by SEQ ID NO: 2) corresponds to the amino acids 1-57 of the amino acid sequence of SEQ ID NO: 2. The second extracellular loop (ECL2) of human GPR182 as defined by SEQ ID NO: 2 corresponds to the amino acids 194 to 217 of the amino acid sequence defined by SEQ ID NO: 2.

The skilled person will be able to identify the amino acid sequence corresponding to the N-terminal extracellular domain and the ECL2 of homologs of the human GPR182 from other species, including mouse and rat GPR182 and of further natural GPR182 variants by employing methods known in the art, including topology and/or structure prediction tools available in the art. For example, structural models of the human, murine and rat GPR182 proteins are retrievable from the AlphaFold database (https://alphafold.ebi.ac.uk/search/text/GPR182).

In view of the herein contemplated purposes to inhibit the GPR182 scavenging function in order to thereby provide a local increase of chemokine ligand levels to render these available for interaction with their signaling receptors (e.g., CXCR4 and/or ACKR3 (CXCR7)), it is particularly preferred that the agent of the invention specifically interacts with the GPR182 protein (and thereby inhibits the binding of one or more of its endogenous ligands to the GPR182), while not showing significant binding to other receptors of these ligand(s) (e.g., CXCR4 and/or ACKR3 (CXCR7)), specifically not to those receptors which upon ligand engagement provide signaling function(s) which are known to account for (or to at least contribute to) any therapeutically beneficial effect(s) envisaged herein.

In this context, it is of note that according to the most updated model of the CXCL12-CXCR4 interaction by Stephens *et al.*, the following epitopes of CXCR4 have been identified as being involved in CXCL12 binding: chemokine recognition site (CRS) CRS0.5, CRS1, CRS1.5 and CRS2. Whereas CRS0.5, CRS1 and CRS1.5 are all comprised in the N-terminal extracellular domain of CXCR4, CRS2 is part of the transmembrane domain pocket and ECL2 (see Stephens BS et al., Sci Signal. 2020;13(640):eaay5024). Gustavsson M. *et al.* investigated the structural basis of ligand interaction with ACKR3 (also known as C-X-C chemokine receptor type 7 (CXCR7) or G-protein coupled receptor 159 (GPR159)) and similarly predicted the involvement of the N-terminal extracellular domain and the CRS2 interaction site of ACKR3 in ligand binding (including CXCL12) (see Gustavsson M. etal., Nat Commun. 2017;8:14135).

As the N-terminal extracellular domain as well as the ECL2 of GPR182 are sufficiently different from the N-terminal extracellular domain and the ECL2 of CXCR4 and ACKR3 (CXCR7), respectively, no significant cross-reactivity of an agent of the invention that specifically binds to GPR182 with respect to a binding to CXCR4 and/or ACKR3 (CXCR7) is expected. For example, a sequence comparison between human GPR182 and human CXCR4 reveals only poor amino acid sequence identities of 14% and 12.5% with respect to their N-terminal extracellular regions (amino acid residues 1-57) and their ECL2, respectively. In a similar vein, a sequence comparison of human GPR182 with human ACKR3 (CXCR7) reveals only low sequence identities of 12.3% and 16.7% in their N-terminal extracellular regions (amino acid residues 1-57) and their ECL2, respectively. An agent that specifically interacts with the N-terminal extracellular domain of GPR182 or with the ECL2 of GPR182 is hence not expected to also possess a significant binding affinity to these or other portions of CXCR4 or ACKR3 (CXCR7). In any case, the skilled artisan will be able to assess the binding of any candidate agent to GPR182, CXCR4, ACKR3 (CXCR7) or other receptors using methods known in the art and described herein, and thus, if desired, to only select such agents having favorable binding activity (and/or which only possess significant binding activity) towards GPR182.

In a recent study by the inventors (Le Mercier *et al.,* 2021), the chemokines CXCL10, CXCL12 and CXCL13 were identified as endogenous (i.e., cognate) ligands of human GPR182. Saturation binding experiments using fluorescently labeled chemokine ligands in connection with HEK293 cells recombinantly expressing human GPR182 revealed that human GPR182 is bound by CXCL10 and CXCL12 with a K_{D} of 19 nM and 41 nM, respectively. Moreover, competition binding experiments revealed inhibition constants (Kᵢ) for CXCL10 and CXCL13 of 9 nM and 10 nM, respectively, and for two CXCL12 isoforms α and β a slightly lower affinity with Ki values of 31 nM and 19 nM, respectively. Although the assessment of other chemokine ligands revealed some interaction with CXCL19 and CXCL16, the binding affinities of these were determined to be much lower (Kᵢ: 260 nm) or even below the limit to be analyzed, respectively, indicating the absence of a physiological relevance of the latter two chemokines on GPR182-mediated functions.

In view of the inventors' findings in connection with the present invention, the detected increased levels of CXCL12 in heart tissue are expected to account for the observed advantageous therapeutic effects in MI. It is hence preferentially understood, in view of the purposes contemplated herein, that the agent which specifically binds to the GPR182 protein has a binding affinity to the GPR182 protein which is at least equal to, or which more preferably exceeds, the binding affinity of the CXCL12 to the GPR182 protein. The skilled person will be able to assess and compare the respective binding affinities of any candidate agent and CXCL12 (or other endogenous ligands) towards the GPR182 protein by employing methods known in the art and some of which are also described herein (see, e.g., Example 2).

Accordingly, in a particularly preferred embodiment, the agent that specifically binds to the GPR182 protein: (i) exhibits a higher binding affinity, preferably an at least 1.5-fold, 2-fold, 3-fold, 4-fold, 5-fold, 6-fold, 7-fold, 8-fold, 9-fold, 10-fold or higher binding affinity, towards the GPR182 protein as compared to the binding affinity between the one or more endogenous GPR182-ligands and the GPR182 protein; and/or (ii) binds to the GPR182 protein with a dissociation constant (K_{D}) which is equal to or less than 10⁻⁶ M, 10⁻⁷ M or 10⁻⁸ M, preferably less than 41 nM, more preferably less than 10⁻⁹ M or 10⁻¹⁰ M.

It is considered that the therapeutic benefits provided by the herein disclosed medical applications that rely on the inhibition of the expression or activity of the GPR182 protein may be even enhanced by a simultaneous and/or supplementary application of one or more therapeutic means that are currently available and routinely employed in the herein envisaged pathological conditions. Respective embodiments are thus also particularly contemplated herein, and some of which are exemplified, yet without being intended to be limiting, in the following.

According to one particular embodiment of the first aspect of the invention, the agent is employed for use in the treatment or prevention of myocardial infarction (MI) and/or one or more post-complication(s) thereof. In preferred embodiments of the latter embodiment, a subject (e.g., a subject at risk for myocardial infarction or a patient diagnosed with a myocardial infarction) is to be administered with a therapeutically effective amount of an agent as disclosed herein and in addition (i.e., in parallel or before or thereafter) be subjected to further therapeutic measures that may enhance the therapeutic effect. For example, a patient diagnosed with myocardial infarction may, in addition to being administered with an agent according to the first aspect of the invention, be subjected to a percutaneous coronary intervention (*i.e.*, a non-surgical procedure that uses a catheter to place a stent to open up blood vessels in the heart which have been narrowed by plaque build-up) and/or to surgical procedures such as coronary artery bypass grafting (CAB); and/or the agent may be administered with one or more further agents, including those that are currently routinely employed in MI therapy. Preferred further agents to that end are defined in the following exemplified embodiment. It is, however, understood that the following embodiment may alternatively also be applied in connection with uses of the agent for the treatment or prevention of any other pathological condition(s) contemplated herein.

In accordance with a preferred embodiment, the agent is to be administered in combination, or sequentially, with one or more of:
(i) a thrombolytic drug; preferably selected from:
   - tissue plasminogen activator; preferably alteplase, retaplase, and/or tenecteplase;
   - streptokinase;
   - anistreplase; and
   - urokinase;
(ii) an anticoagulant; preferably selected from:
   - a heparin; preferably unfractionated heparin (UFH) and/or low molecular weight heparin (LMWH);
   - a factor Xa inhibitor; preferably apixaban and/or rivaroxaban;
      and
   - hirudine and/or bivalirudin;
(iii) a platelet agglutination inhibitor; preferably selected from:
   - an irreversible cyclooxygenase inhibitor; preferably acetylsalicylic acid (ASA) and/or triflusal;
   - an adenosine diphosphate (ADP) receptor inhibitor; preferably cangrelor, clopidogrel, pasugrel, ticagrelor and/or ticlopidine;
   - a phosphodiesterase inhibitor; preferably cilostazol;
   - a protease-activated receptor-1 (PAR-1) antagonist; preferably vorapaxar,
   - a glycoprotein IIB/IIIA inhibitor; preferably abciximab, eptifibatide, and/or tirofiban;
   - an adenosine reuptake inhibitor; preferably dipyridamole;
   - a thromboxane inhibitor;
   - a thromboxane synthase inhibitor;
   - a thromboxane receptor antagonist; preferably terutroban;
(iv) a beta blocker; preferably selected from propranolol, alprenolol, bucindolol, carteolol, carvedilol, labetalol, levobunolol, medroxalol, mepindolol, metipranolol, nadolol, oxprenolol, penbutolol, pindolol, sotalol, timolol, acebutolol, atenolol, betaxolol, bisoprolol, celiprolol, esmolol, metoprolol, and nebivolol; and
(v) an angiotensin-converting-enzyme inhibitor (ACE inhibitor); preferably selected from benazepril, zofenopril, perindopril, trandolapril, captopril, enalapril, lisinopril, and ramipril;
(vi) a nitrate; preferably isosorbide dinitrate, isosorbide mononitrate and/or pentaerythritol tetranitrate;
(vii) oxygen;
(viii) one or more C-X-C chemokine receptor type 4 (CXCR4) agonist(s), preferably CXCL12 and/or one or more synthetic CXCR4 agonist(s);
(ix) one or more C-X-C motif chemokine receptor type 7 (CXCR7) agonist(s), preferably CXCL11, CXCL12 and/or one or more synthetic CXCR7 agonist(s);
(x) a C-X-C motif chemokine ligand 10 (CXCL10); and/or
(xi) a C-X-C motif chemokine ligand 13 (CXCL13);
and wherein preferably the pathological condition is selected from a myocardial infarction (and/or one or more post-complications thereof) and stroke.

According to one particular embodiment of the first aspect of the invention, the agent is employed for use in the treatment or prevention of a cancer. In exemplary embodiments of the latter embodiment, a subject (e.g., a subject at risk for developing a cancer or patient afflicted with a cancer) is to be administered with a therapeutically effective amount of an agent as disclosed herein and may in addition (*i.e.*, in parallel or before or thereafter) be subjected to a radiotherapy and/or a surgical procedure for removal of cancerous cells or tissues, and/or the agent may be administered in combination with one or more further anti-cancer agents, such as, without limitation, one or more chemotherapeutic agents, and/or in combination with an immunotherapy. Preferred agents/therapies to be combined with the agents of the invention are defined in the following exemplified embodiment. It is, however, understood that the following embodiment may alternatively also be applied in connection with uses of the agent for the treatment or prevention of any other pathological conditions contemplated herein.

Thus, according to preferred embodiments, the agent is to be administered in combination, or sequentially, with one or more of:
(i) one or more chemotherapeutic agent(s);
(ii) an adoptive cellular therapy, preferably an adoptive T cell therapy;
(iii) one or more immune-checkpoint-inhibitors, preferably selected from an antibody directed against cytotoxic T-lymphocyte-associated antigen 4 (CTLA4), programmed death 1 (PD-1), programmed death ligand 1 (PD-L1) and programmed death ligand 2 (PD-L2) or combinations thereof; and/or
(iv) one or more C-X-C motif chemokine receptor type 3 (CXCR3) agonist(s), preferably CXCL9, CXCL10, or CXCL11 and/or one or more synthetic CXCR7 agonist(s);
and wherein preferably the pathological condition is a cancer, more preferably a melanoma.

The term "chemotherapeutic agent", as used herein, refers to a compound that can be useful in the treatment of a disease (e.g., cancer). Exemplary chemotherapeutic agents effective against cancer include, without limitation, daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, taxotere, vincristine, vinblastine, etoposide, teniposide, cisplatin and diethylstilbestrol (DES).

The term "immunotherapy", as used herein, refers generally to means for treatment of a disease or condition (e.g., a cancer) by inducing, enhancing, or suppressing an immune response. The term "immunotherapy" also includes adoptive cellular therapies.

For purpose of the present disclosure, the terms "adoptive cellular therapy (ACT)" or "cellular adoptive immunotherapy" or "adoptive immunotherapy" refer to a cell-based therapy with various lymphocytes and/or antigen-presenting cells. For example, in some instances, the therapy involves collecting T cells from a patient and growing the collected T cells in the laboratory to increase the number of T cells. These T cells are then given back to the same patient or another to help the immune system fight disease, e.g., a cancer. Moreover, the term "adoptive cellular therapy (ACT)", as used herein also means an immunotherapy in which (a) genetically modified lymphocytes are administered to a patient or (b) targeted gene therapy vectors that modify lymphocytes are administered to a patient.

Adoptive cellular therapies include, but are not limited to, CAR-T therapies.

For purposes of the present disclosure, the term "CAR T therapy" or "CAR T cell therapy" refers to treating a disease (e.g., a cancer) using CAR T cells. For purposes of the present disclosure, the term "CAR-T" or the term "CAR T cell" refers to a genetically engineered T cell that produces a "chimeric antigen receptor (CAR)" on the surface of the cell. Chimeric antigen receptors (CARs, also known as chimeric immunoreceptors, chimeric T cell receptors or artificial T cell receptors) are receptor proteins that have been engineered to give T cells the new ability to target a specific protein. The receptors are chimeric because they combine both antigen-binding and T cell activating functions into a single receptor. The backbone of a CAR-T or CAR T cell is a T cell, which is often collected and separated from a subject, such as a patient, who is going to receive the CAR-T or CAR T cell. A CAR T cell is usually an engineered patient's immune cell used to treat the patient's cancer. For example, a CAR T therapy may be an immunotherapy utilizing a subject or a patient's own immune cells that are engineered to be able to produce a particular chimeric antigen receptor(s) on their surface. In some situations, T cells are collected from the body of a subject or a patient via apheresis, a process that withdraws blood from the body and removes one or more blood components (such as plasma, platelets or white blood cells). The T cells collected from the body are then genetically engineered to produce a particular chimeric antigen receptor on their surface. After the engineering, the T cells are known as chimeric antigen receptor (CAR) T cells. The CAR T cells are expanded by growing in a laboratory and then administered to the subject or patient, or another subject or patient. The CAR T cells will recognize and kill cancer cells that express a targeted antigen on their surface. For purposes of the present disclosure, the term "chimeric antigen receptor" and the term "CAR" refers to an engineered receptor that recognize a targeted antigen expressed on the surface of a target cell, such as an antigen expressed on a cancer cell.

As used herein the term "immune checkpoint protein" has its general meaning in the art and refers to a molecule that is expressed by T cells and/or NK cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see, e.g., Pardoll, 2012. Nature Rev Cancer 12:252-264; Mellman et al., 2011. Nature 480:480-489). Examples of inhibitory checkpoint molecules include B7-H3, B7-H4, BTLA, CTLA-4, CD277, KIR, PD-1, LAG-3, TIM-3, TIGIT and VISTA. B7-H3, also called CD276, was originally understood to be a co-stimulatory molecule but is now regarded as co-inhibitory. B7-H4, also called VTCN1, is expressed by tumor cells and tumor-associated macrophages and plays a role in tumor escape. B and T Lymphocyte Attenuator (BTLA), also called CD272, is a ligand of HVEM (Herpesvirus Entry Mediator). Cell surface expression of BTLA is gradually downregulated during differentiation of human CD8+ T cells from the naive to effector cell phenotype, however tumor-specific human CD8+ T cells express high levels of BTLA. Cytotoxic T-Lymphocyte-Associated protein 4 (CTLA-4), also called CD152 is overexpressed on Treg cells serves to control T cell proliferation. Killer-cell Immunoglobulin-like Receptor (KIR) is a receptor for MHC Class I molecules on Natural Killer cells. Lymphocyte Activation Gene-3 (LAG3) works to suppress an immune response by action to Tregs as well as direct effects on CD8+ T cells. T-cell Immunoglobulin domain and Mucin domain 3 (TIM-3) expresses on activated human CD4+ T cells and regulates Th1 and Th17 cytokines. TIM-3 acts as a negative regulator of Th1/Tc1 function by triggering cell death upon interaction with its ligand, galectin-9. V-domain Ig suppressor of T cell activation (VISTA) is primarily expressed on hematopoietic cells so that consistent expression of VISTA on leukocytes within tumors may allow VISTA blockade to be effective across a broad range of solid tumors. Programmed cell death protein 1 (PD-1), also known as CD279, acts as an immune checkpoint, which upon binding of one of its ligands, programmed cell death ligand 1 (PD-L1) or programmed cell death ligand 2 (PD-L2), enables Shp2 to dephosphorylate CD28 and inhibits the activation of T cells.

As used herein, the term "immune checkpoint inhibitor" has its general meaning in the art and refers to any compound inhibiting the function of an immune inhibitory checkpoint protein. Inhibition in this context includes reduction of function and full blockade. In particular, the immune checkpoint inhibitor particularly suitable for enhancing the proliferation, migration, persistence and/or cytotoxic activity of CD8+ T cells in the patient and in particular the tumor-infiltrating of CD8+ T cells of the patient. As used herein, the term "CD8+ T cell" has its general meaning in the art and refers to a subset of T cells which express CD8 on their surface. They are MHC class I-restricted, and function as cytotoxic T cells. "CD8+ T cells" are also called cytotoxic T lymphocytes (CTL), T-killer cells, cytolytic T cells, or killer T cells. CD8 antigens are members of the immunoglobulin supergene family and are associative recognition elements in major histocompatibility complex class I-restricted interactions. As used herein, the term "tumor infiltrating CD8+ T cell" refers to the pool of CD8+ T cells of the patient that have left the blood stream and have migrated into a tumor.

Non-limiting examples of other anti-cancer agents include but are not limited to, angiogenesis inhibitors, antiproliferative agents, other kinase inhibitors, other receptor tyrosine kinase inhibitors, aurora kinase inhibitors, polo-like kinase inhibitors, bcr-abl kinase inhibitors, growth factor inhibitors, antimitotic agents, alkylating agents, antimetabolites, platinum containing agents, growth factor inhibitors, ionizing radiation, cell cycle inhibitors, topoisomerase inhibitors, biologic response modifiers, immunomodulators, immunologicals, antibodies, hormonal therapies, retinoids/deltoids plant alkaloids, proteasome inhibitors, HSP-90 inhibitors, histone deacetylase inhibitors (HDAC) inhibitors, purine analogs, pyrimidine analogs, MEK inhibitors, CDK inhibitors, ErbB2 receptor inhibitors, mTOR inhibitors, Bcl inhibitors, Mcl inhibitors and combinations thereof as well as other antitumor agents. Angiogenesis inhibitors include, but are not limited to, EGFR inhibitors, PDGFR inhibitors, VEGFR inhibitors, TIE2 inhibitors, IGFIR inhibitors, matrix metalloproteinase 2 (MMP-2) inhibitors, matrix metalloproteinase 9 (MMP-9) inhibitors, thrombospondin analogs such as thrombospondin-1.

In another preferred embodiment, the agent that specifically binds to the GPR182 protein is a (poly)peptide and is to be administered in the form of a polynucleotide encoding said (poly)peptide; wherein more preferably the polynucleotide is:
(i) comprised in a vector; preferably in a viral vector, more preferably in an adeno-associated virus (AAV) vector; or
(ii) an RNA, preferably an mRNA;

In preferred embodiments, the agent does not possess substantial cytotoxic and/or immunogenic activity.

In preferred embodiments, the agent that specifically binds to the GPR182 protein is an antibody or Fc fusion polypeptide and does not cause substantial antibody-dependent cellular cytotoxicity (ADCC) and/or complement-dependent cytotoxicity (CDC).

In preferred embodiments of the first aspect of the invention, or in further aspects of the invention, the agent as defined herein may be employed for use in:
(i) promoting tissue repair, preferably angiogenesis and/or vascularisation, after (acute) myocardial infarction;
(ii) reducing infarction size and/or volume after (acute) myocardial infarction;
(iii) enhancing the perfusion and/or reducing the risk for or severity of heart insufficiency, myocardial necrosis, cardiac hypertrophy, cardiac fibrosis, limb ischemia, ischemia-related tissue degeneration, and/or ventricular arrhythmias after (acute) myocardial infarction;
(iv) promoting angiogenesis and/or vascularisation in an ischemic disease;
(v) enhancing anti-tumor immunity;
(vi) enhancing the tumor-infiltration by T cells, preferably CD8+ cytotoxic T cells and/or CAR T cells; and/or;
(vii) enhancing the efficacy of adoptive cellular therapy.

In a further preferred embodiment, the agent as defined herein is for use in treating myocardial infarction by promoting the formation of collateral coronary arteries.

In preferred embodiments, the agent that specifically binds to the GPR182 protein is:
(i) distinct from CXCL10, CXCL12, and/or CXCL13;
(ii) distinct from CXCL9, CXCL10, and/or CXCL11;
(iii) distinct from CXCL9, CXCL10, CXCL11, CXCL12, and/or CXCL13; and/or
(iv) heterologous to the subject which is to be administered with the agent.

It is understood by the skilled artisan that for the medical uses contemplated herein, the agent is to be administered to a subject in a therapeutically effective amount.

The term "therapeutically effective amount", "pharmaceutically effective amount" or "effective amount" as interchangeably used herein, refers to an amount, which has a therapeutic effect or is the amount required to produce a therapeutic effect in a subject. For example, a therapeutically effective amount of an agent or a pharmaceutical composition thereof is the amount of the agent or the pharmaceutical composition required to produce a desired therapeutic effect as may be judged by clinical trial results, model animal studies, and/or *in vitro* studies. The pharmaceutically effective amount depends on several factors, including but not limited to, the specific pathological condition that is targeted, characteristics of the subject (for example weight, gender, age, and medical history), and the particular type of the agent used. For prophylactic (i.e., preventive) application (or treatment), a therapeutically or prophylactically effective amount is that amount which would be effective in preventing a respective pathological condition or of not yet occurring disease symptoms or further complications which may result from that pathological condition.

The agent may be formulated for administration according to the invention as part of a pharmaceutical composition. Pharmaceutical compositions preferably comprise a pharmaceutically acceptable carrier or excipient. By "pharmaceutically acceptable carrier or excipient" is meant a non-toxic solid, semi-solid or liquid filler, diluent, encapsulating material or formulation auxiliary of any type (see also Handbook of Pharmaceutical Excipients 6ed. 2010, Published by the Pharmaceutical Press). Examples of suitable pharmaceutical carriers and excipients are well known in the art and include phosphate buffered saline solutions, water, emulsions, such as oil/water emulsions, various types of wetting agents, sterile solutions, organic solvents including DMSO etc. Compositions comprising such carriers or excipients can be formulated by well-known conventional methods. The pharmaceutical composition may be administered, for example, orally, parenterally, such as subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable carriers or excipients.

For oral administration, the agent or pharmaceutical composition comprising said agent may be formulated into solid dosage forms such as tablets, pills, powders, granules or capsules, etc., and these solid dosage forms may be prepared by mixing the agent of the invention with one or more excipients such as starch, calcium carbonate, sucrose or lactose, gelatine, etc. Moreover, lubricants such as magnesium stearate, talc, etc. can be used in addition to simple excipients. Furthermore, the pharmaceutical composition may be formulated into liquid dosage forms such as suspensions, liquid for internal use, emulsions, syrups, etc., and various excipients such as humectants, sweeteners, aromatics, preservatives, etc. in addition to water and liquid, paraffin may be used for the formulation of liquid dosage forms. For parenteral administration, the pharmaceutical composition is formulated generally by mixing the agent of the invention at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) peptides, e.g., polyarginine or tripeptides; serum albumin, gelatin; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The agent according to the invention (or pharmaceutical composition comprising said agent) can be administered systemically (e.g., orally, rectally, parenterally (e.g., intravenously), intramuscularly, intraperitoneally, transdermally (e.g., by a patch), topically (as by powders, ointments, drops or transdermal patch), bucally, or as an oral or nasal spray, by inhalation, subcutaneously or the like), by administration into the central nervous system (e.g., into the brain (e.g., intracerebrally, intraventricularly or intracerebroventricular) or spinal cord or into the cerebrospinal fluid), or any combination thereof.

In preferred embodiments, the agent or the polynucleotide encoding the agent is to be administered intravenously, intramuscularly and/or orally.

The agent or pharmaceutical composition comprising said agent may be administered in one or more dose administrations, at least once daily, for one or more days (e.g., 1, 2, 3, 4, 5, 6, 7 days; 2, 3, 4 weeks; 2, 3, 4, 5, 6, 7, 8, 9, 10, 11, 12 months; 2 years, 3 years, 4 years, or longer). However, the skilled person understands that the duration of the administration, in accordance with the present invention, can optionally be sustained for the remaining lifetime of the subject. The agent or pharmaceutical composition comprising said agent may be administered daily or less frequently, for example, every-other-day, semi-weekly, weekly, biweekly, semi-monthly, monthly, bimonthly, etc.

The agent of the invention (or pharmaceutical composition comprising said agent) can be administered to the subject at a suitable dose and/or a therapeutically effective amount.

The dosage of the agent of the invention required will somewhat vary from subject to subject, depending on the species, age, weight, and general condition of the subject, the particular active agent and pharmaceutical carrier used, the mode of administration and the like. For example, the dose of the agent when administered parenterally may be in the range of about 0.01 mg/kg to 1000 mg/kg of patient body weight (bd wt), although, as noted above, this will be subject to therapeutic discretion. More preferably, this dose is at least 0.5 mg/kg/day, and most preferably for humans between about 5 and 100 mg/kg/day. If given continuously, the pharmaceutical composition is typically administered at a dose rate of about 1 mg/kg/hour to about 50 mg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

For determining therapeutically effective dosages of the agent or particular pharmaceutical formulations comprising the agent, for example, animal models (e.g., mice) expressing the human GPR182 protein can be used to monitor levels of response. Exemplary methods that may also be utilized for identifying suitable dosages and administration regimens are those described in connection with the fourth and fifth aspect of the invention.

Moreover, the present invention relates in a third aspect to an *in vitro* method for identifying an agent suitable for the use according to the first aspect of the invention, the method comprising assessing, in the presence of a candidate agent, the binding of one or more endogenous GPR182-ligands to cells expressing the GPR182 protein, wherein a reduced binding of the one or more endogenous GPR182-ligands in the presence of the candidate agent as compared to in the absence of the candidate ligand indicates that the candidate agent is suitable for the use according to the first aspect of the invention.

In preferred embodiments of the method according to the third aspect of the invention, either the endogenous ligand or the candidate agent is fluorescently labelled, and the binding is assessed by means of assessing the fluorescent signal in a fluorescent competition assay; and/or the cells expressing the GPR182 protein are recombinant eukaryotic cells; more preferably HEK293T cells.

Moreover, the present invention relates in a fourth aspect to a method for evaluating the efficacy of an agent as defined in any of the preceding claims in the treatment of a myocardial infarction, comprising assessing before, and/or simultaneously with, and subsequent to an administration of the agent one or more of the following:
(a) the size or volume of the infarction area;
(b) the left ventricular ejection fraction and/or left ventricular end-diastolic volume, and/or left ventricular end-systolic volume;
(c) the concentration of CXCL12 in the heart and/or blood; and/or
(d) the plasma level(s) of one or more biological markers of myocardial damage, wherein the biological marker is preferably troponin-I and/or troponin-T;
whereby a decrease of the volume of the infarction area, an improvement of the left ventricular ejection fraction, a reduction of the left ventricular end-diastolic volume, a reduction of the left ventricular end-systolic volume, an increase of the CXCL12 concentration in the heart and/or blood, and/or a decrease of the plasma level(s) of the one or more biological markers of myocardial damage determined subsequent to the administration of the agent provides an indication of the agent being efficacious in the treatment of myocardial infarction.

Means and methods for determining the size or volume of the infarction area are known and routinely employed in the art, such as echocardiography as well as by magnetic resonance imaging (MRI) as used in the appended examples (see also, e.g., Lee DC et al. J Am Heart Assoc. 2020;9(3):e014205). Other approaches routinely used in the art for these purposes are based on contrast enhanced computed tomography (CECT) (see, e.g., textbook "Computed Tomography of the Cardiovascular System" by Thomas C. Gerber, Birgit Kantor, Eric E. Williamson, CRC Press; 1st Ed. 2007).

The left ventricular ejection fraction and/or left ventricular end-diastolic volume, and/or left ventricular end-systolic volume can be determined by using multiple noninvasive imaging modalities, including echocardiography, cardiac magnetic resonance (CMR) imaging, and gated single-photon emission computed tomography (SPECT) imaging. All of these methods are routinely used for clinical decision making as well as research study enrollment.

Moreover, the present invention relates in a fifth aspect to a method for evaluating the efficacy of an agent as defined in any of the preceding claims in the treatment of a cancer, comprising assessing before, and/or simultaneously with, and subsequent to an administration of the agent to a subject one or more of the following:
(a) the level of CXCL9 and/or CXCL10 in the cancer and/or blood;
(b) the level of T cells, preferably CD8+ cytotoxic T cells, in the cancer and/or blood; and/or
(c) the size of the cancer;
whereby an increase of the CXCL9 and/or CXCL10 concentration in the cancer and/or the blood, and/or an increase of the level of T cells, preferably CD8+ cytotoxic T cells, in the cancer and or blood, and/or a decrease of the size of the cancer determined subsequent to the administration of the agent provides an indication of the agent being efficacious in the treatment of the cancer.

The present invention relates in a sixth aspect to a method of treating and/or preventing any of the pathological condition(s) as referred to herein in connection with the medical uses according to the first or other aspect(s) of the invention, the method comprising administering a therapeutically effective amount of the agent to a subject in need thereof.

It is understood that the definitions and embodiments as described above in the context of the first aspect of the invention also apply, in as far as possible, *mutatis mutandis* to the second, third, fourth fifth and sixth, as well as to any further disclosed aspects of the present invention.

The invention is herein described, by way of example only, with reference to the accompanying drawings for purposes of illustrative of the preferred embodiments of the present invention.

The Figures show
**Fig. 1****:** Expression of GPR182 in vascular endothelial cells (VEC) and lymphatic endothelial cells (LEC) in the heart before and at the indicated days after myocardial infarction. Shown is the percentage of endothelial cells expressing GPR182. Data are based on a further analysis conducted by the inventors of the transcriptome profiling data set available from Tombor *et al.* (2021).
**Fig. 2****:** Size of infarct 3 weeks after myocardial infarction in mice. (**A, C**) Masson Trichrome-stained sections of the heart from control and GPR182-deficient mice (Gpr182 gKO) (**A**) or from animals with induced endothelium-specific GPR182 deficiency (EC-Gpr182-KO) (**C**) 3 weeks after myocardial infarction. Serial sections were generated at a distance of 600 µm starting from the site of the ligation to the basis or the apex, respectively (**B, D**). Quantification of the infarct size in the sections is shown in **A** and **C.** Shown are mean values ± SEM; *, P ≤ 0.05.

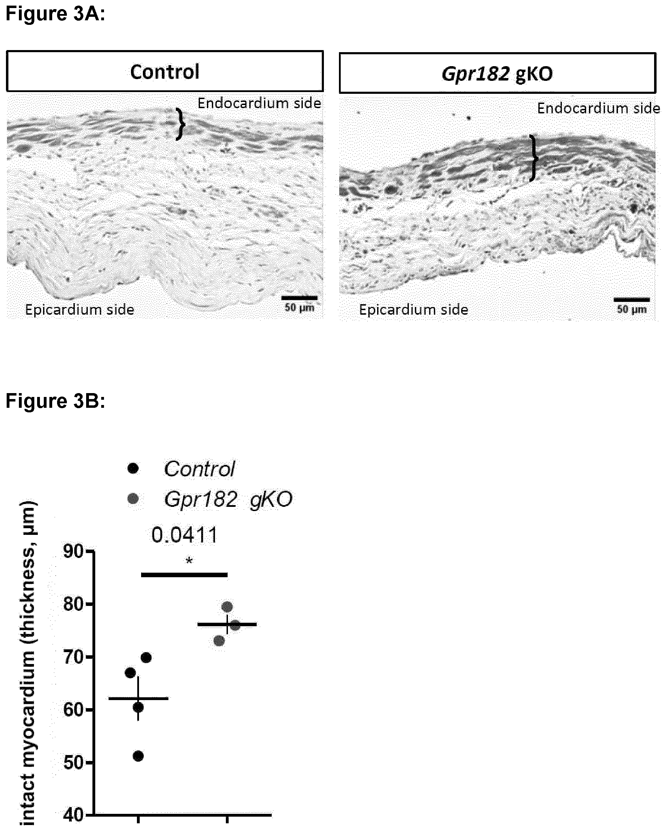

**Fig. 3****:** Thickness of intact myocardium in infarct area. (**A**) H&E-stained sections of the heart from control and GPR182-deficient mice (Gpr182gKO) 3 weeks after myocardial infarction. Brackets indicate intact myocardium. (**B**) Statistical analysis of the thickness of the intact myocardium in the infarct area. Shown are mean values ± SEM; *, P ≤ 0.05.

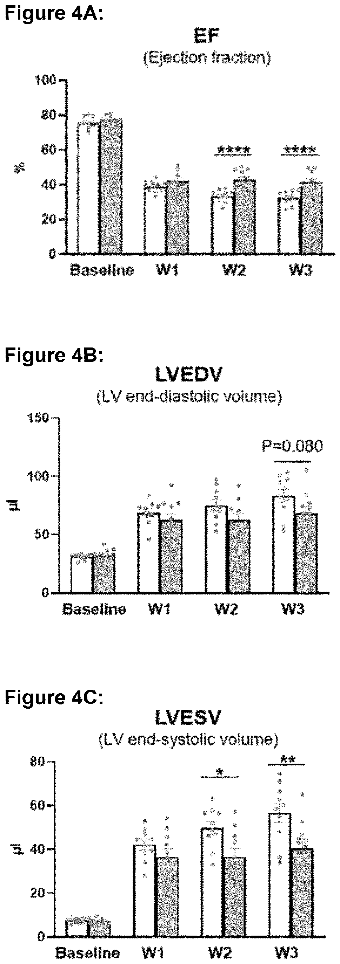

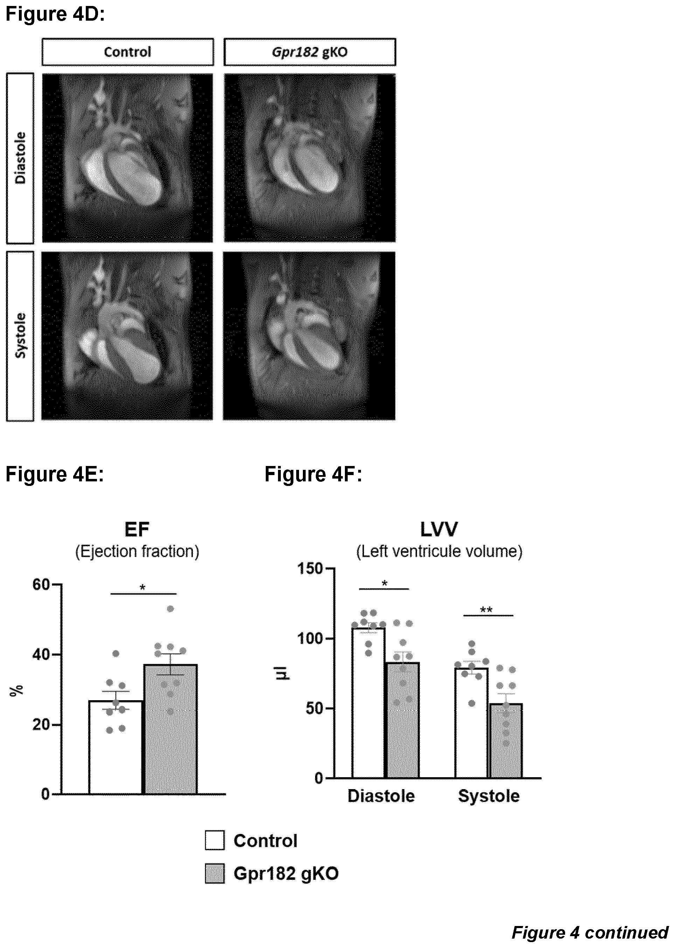

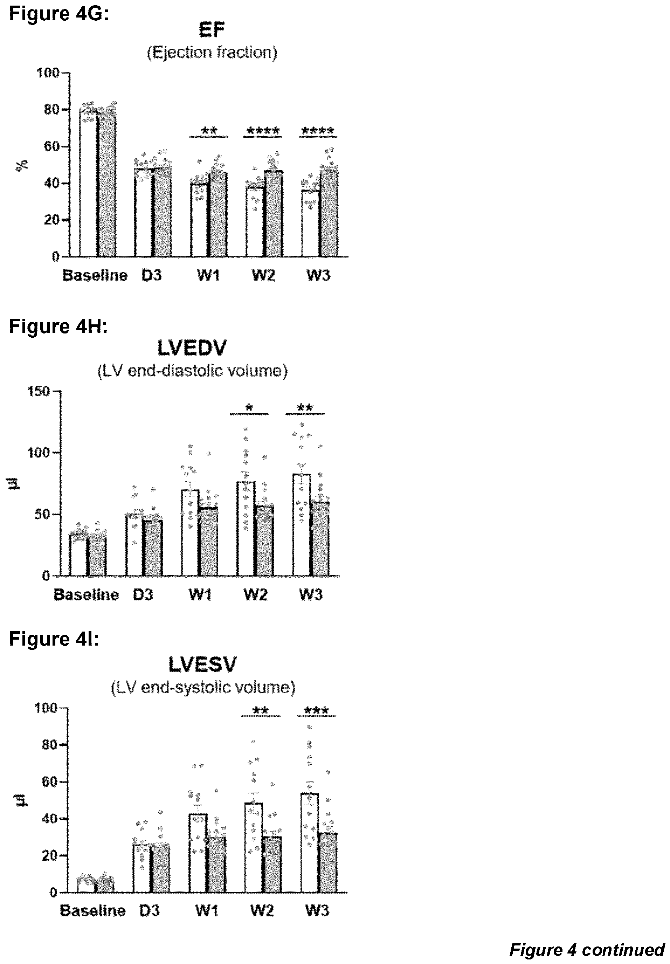

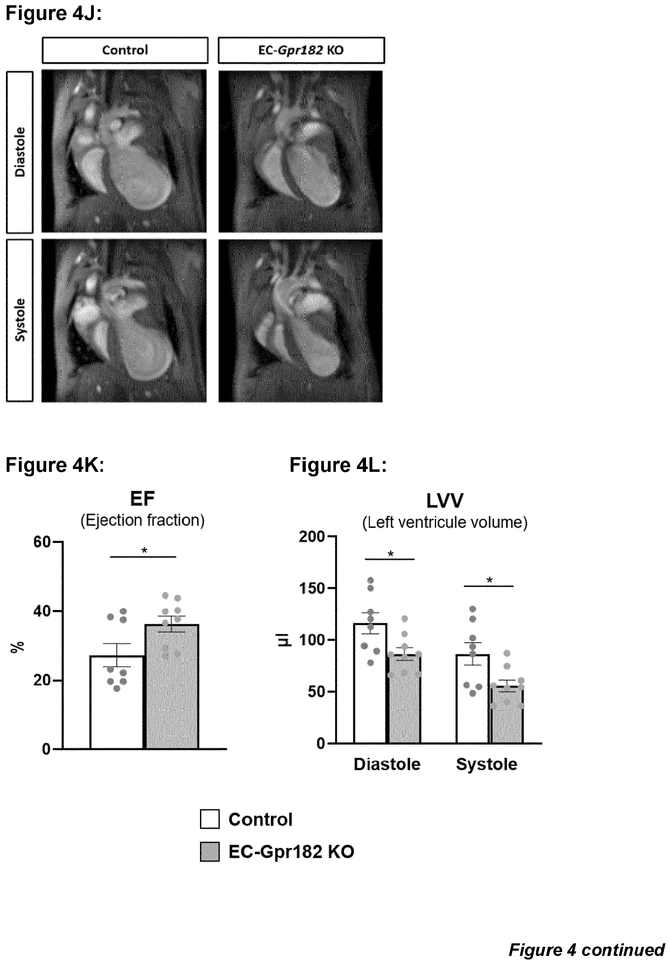

**Fig. 4****:** Functional and morphological changes after myocardial infarction in wild-type and GPR182-deficient mice analyzed by echocardiography or cardiac MRI. (**A-C**, **G-I**) Ejection fraction (EF), left ventricular end-diastolic volume (LVEDV) and left ventricular end-systolic volume (LVESV) were determined by echocardiography in control animals and GPR182-deficient mice (Gpr182gKO) (**A-C**) or in animals with endothelium-specific GPR182- deficiency (EC-Gpr182-KO) (**G-I**) before (baseline) and at the indicated time points after myocardial infarction. (**D**, **J**) Representative images of cardiac MRI 3 weeks after myocardial infarction in control animals as well as in Gpr182gKO (**D**) or EC-Gpr182-KO (**J**) animals. (**E**, **F**, **K**, **L**) Ejection fraction (EF) and left ventricular volume (LVV) 3 weeks after myocardial infarction determined by cardiac MRI in control animals as well as in Gpr182gKO (**E**, **F**) or EC-Gpr182-KO (**K, L**) animals. Shown are mean values ± SEM; *, P ≤ 0.05; **, P ≤ 0.01; ***, P ≤ 0.001; ****, P ≤ 0.0001.

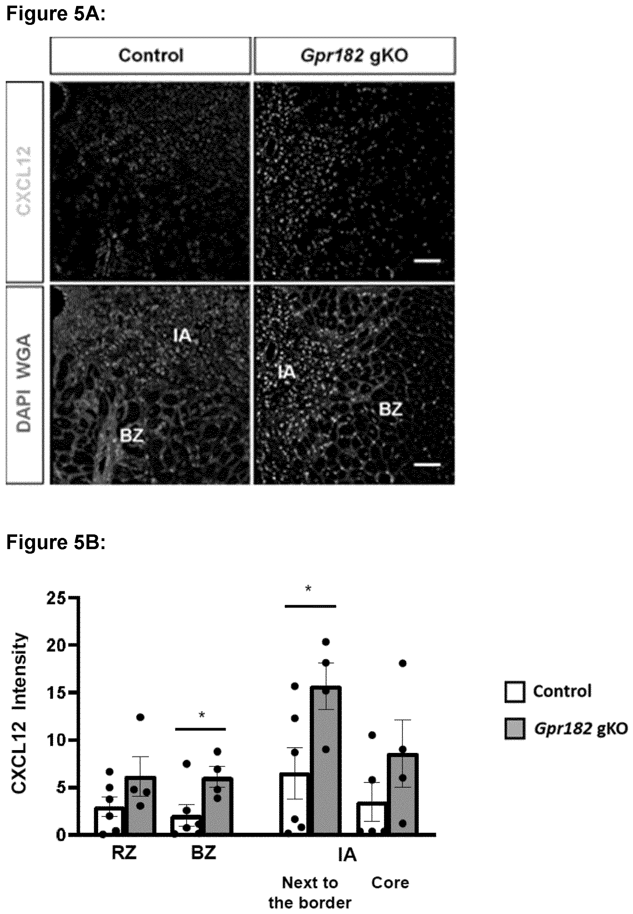

**Fig. 5****:** Local protein levels of CXCL12 in ischemic hearts. Hearts were analyzed 3 days after ligation and paraffin sections were stained with an anti-CXCL12 antibody. Nuclei and membranes were stained by DAPI or Wheat germ agglutinin (WGA). (**A**) Representative images of the infarct border zone in Gpr182gKO animals as well as in control mice. Scale bar: 50 µm. (**B**) Quantification of the local CXCL12 levels in the remote zone (RZ) in the infarct zone (IA) as well as in the border zone (BZ) of the infarct. Shown are mean values ± SEM; *, P ≤ 0.05.

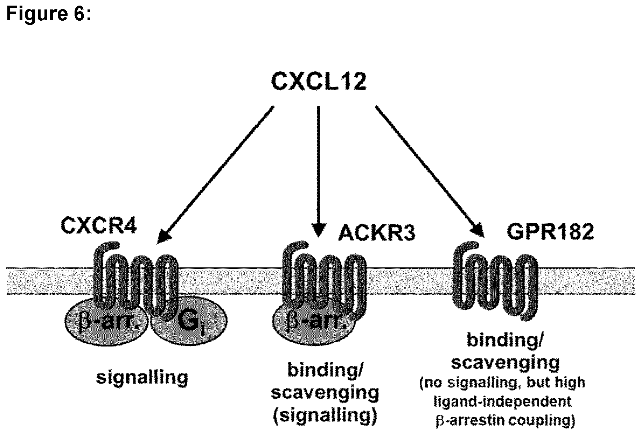

**Fig. 6**: A schematic showing CXCL12 and its three receptors: CXCR4, the main mediator of CXCL12 effects, which signals through β-arrestin as well as Gi-type proteins, the atypical chemokine receptor ACKR3 (CXCR7), which binds and scavenges CXCL12 but can also signal through β-arrestin albeit not through G-proteins, as well as GPR182, which is solely a binding/scavenging receptor without any signaling properties.

The examples illustrate the invention:

### Example 1: Inhibition of the GPR182-mediated chemokine ligand scavenging improves clinical outcome in an animal model of acute myocardial infarction (MI)

### 1.1 METHODS

### Animal models

All mice were backcrossed onto a C57BL/6J background at least 8 to 10 times, and experiments were performed with littermates as controls. Mice were housed under a 12-hour light-dark cycle, with free access to food and water and under specific pathogen-free conditions unless stated otherwise.

Mice lacking GPR182 (Gpr182 gKO) and mice with an endothelial specific inducible GPR182 deficiency (EC-Gpr182-KO) were generated as previously described (Le Mercier *et al.,* 2021). For induction of Cre-mediated recombination in EC-Gpr182-KO mice (*Cdh5CreERT2, Gpr182*^{*f*/*f*}), 1 mg of tamoxifen diluted in Myglyol was injected intraperitoneally for 5 consecutive days in mice aged between 8 to 10 weeks. Experiments were performed 2 weeks after the last tamoxifen injection.

### Myocardial infarction

Coronary ligation was performed in male mice at an age of 10 to 12 weeks. Briefly, following anesthetization (isoflurane inhalation) and tracheal intubation, the chest cavity was opened from the 4^{th} intercostal space. The left ascending coronary artery was ligated tightly with a 8-0 suture under microscopic control. Myocardial ischemia was confirmed by observing the color changes in the segment of the left ventricle subjected to coronary flow occlusion. The wound was closed with a 5-0 suture.

After surgery, mice were monitored every day for 3 days, and treated with an intraperitoneal injection of buprenorphine (0.1 mg/kg) twice a day. Mice that died within 24 hours of surgery were excluded from analysis. Surgery was performed by an individual who was blinded to the mouse genotype.

### Echocardiography

Cardiac function was assessed before and after surgery at indicated time points using the Vevo 2100 ultrasound system (VisualSonics, Fujifilm) equipped with a MS550d transducer. Left ventricular ejection fraction (LVEF), LV fractional area change, LV systolic and diastolic volume were measured by Simpson's method.

### Magnetic Resonance Imaging

Cardiac MRI measurements were performed on a 7.0 T Bruker Pharmascan, equipped with a 300 mT/m gradient system, using a custom-built circularly polarized birdcage resonator and the Early Access Package for self-gated cardiac Imaging (Bruker, Ettlingen, Germany). The mice were measured under volatile isoflurane (2.0%) anesthesia. The measurement is based on the gradient echo method (repetition time=6.2 ms; echo time=1.6 ms; field of view=2.20α2.20 cm; slice thickness=1.0 mm; matrix=128×128; repetitions=100). The imaging plane was localized using scout images showing the 2- and 4-chamber view of the heart, followed by acquisition in short-axis view, orthogonal on the septum in both scouts. Multiple contiguous short-axis slices consisting of 9 or 10 slices were acquired for complete coverage of the left ventricle. Magnetic resonance imaging data were analyzed using Qmass digital imaging software (Medis, Leiden, Netherlands).

### Histological analysis

Heart tissues were harvested at indicated time points after coronary ligation. Heart tissues were perfused with cold PBS from the apex and fixed in 4% PFA at 4 °C overnight. Samples were embedded in paraffin. 5-µm-thick serial sections of the short axis of the heart were deparaffinized and rehydrated. Masson's trichrome staining was performed according to the manufacturer's instructions (Sigma-Aldrich, HT15). For CXCL12 immunofluorescence staining, after rehydration of tissue sections, endogenous peroxidases were inactivated using 3% H₂O₂ in H₂O for 30 min. Slides were then subjected to microwave-assisted antigen retrieval in a solution of 10 mM sodium citrate, 0.05% tween 20, pH 6.0 for 5 min at 1000 W followed by 10 min at 500 W. After cooling and removal of antigen retrieval solution, sections were blocked and permeabilized in antibody diluent (PBS, horse serum 5%, triton 0.1%) for 1 h at room temperature, followed by avidin/biotin blocking using avidin/biotin blocking kit from Vector Laboratories (SP-2001). Sections were then incubated with anti-SDF1 (CXCL12) (Abcam, ab25117, 1:200) diluted in antibody diluent at 4 °C overnight. After washing, slides were incubated with goat anti rabbit-IgG biotinylated secondary antibody for 30 min at room temperature (Vector Laboratories, PK-6101, 1:500) followed by amplification using VECTASTAIN^{®} Elite ABC-HRP Kit, Peroxidase (Rabbit IgG) (Vector Laboratories, PK-6101) for 30 min at room temperature. The signal was revealed and enhanced with the TSA-Cy3 signal amplification kit (AKOYA BIOSCIENCES, SAT704A001EA). The cell membrane and nuclei were visualized with AF647-conjugated WGA (Thermo Fisher Scientific, W32466, 5 µg/ml) and DAPI (LifeTechnologies D3571), respectively. Sections were mounted in Fluoromount and covered with glass coverslips, and images were acquired by confocal microscopy using Leica TCS SP8 confocal microscope.

### Bioimaqe analysis

All microscopy images were analyzed using Image J or Fiji distribution of ImageJ. Infarct size was calculated as averaged percentage of the scar length to the total LV circumference.

CXCL12 fluorescence intensity was measured using mean signal intensity of hand delineated region of interests corresponding to remote zone, border zone, or infarct. Measurement of healthy myocardium thickness at the endocardial side of the infarct: we manually selected (using selection brush tool) the healthy myocardium area directly adjacent to the endocardium in the infarct region of hematoxylin-eosin (H&E)-stained paraffin sections. This represents the selection #1. A mask was generated from the selection #1, duplicated, and the Local Thickness (complete process) plugin was applied to the duplicated mask. The skeletonize function was applied to the first mask and a new selection (#2) was generated. This selection #2 that represents the medial line along the healthy myocardium selection was used on the output image from Local Thickness plugin to measure the average pixel intensity from the selection #2. This measurement represents the average thickness along the selection #1 (healthy myocardium). Papillary muscles from the left ventricle were excluded from the analysis.

### Statistics

Statistical analysis was performed using the GraphPad Prism software v.8.3.0 from GraphPad Software Inc. (La Jolla, CA, USA). Values are presented as mean ± SEM. Statistical analysis for 2 groups was performed with an unpaired two-tailed Student's t test, or nonparametric Mann-Whitney U test when appropriate. Results of repeated measurements were evaluated by 2-way repeated measure ANOVA with *post hoc* Sidak or Tukey multiple comparison tests. A p value of less than 0.05 was considered to be statistically significant.

### 1.2 INTRODUCTION

GPR182 has been recently revealed to be expressed in endothelial cells of the microvascular and lymphatic system in mammalian species including humans (Le Mercier *et al.*, 2021; Schmid *et al.*, 2018; Torphy *et al.,* 2022; Xiao *et al.,* 2014), and to function as an atypical chemokine receptor, i.e., it binds chemokines with reasonably high affinity. However, ligand binding to GPR182 does not induce any downstream signaling (Le Mercier et al., 2021). At the same time, GPR182 has a relatively high basal constitutive activity towards β-arrestin recruitment (Kroeze et al., 2015; Le Mercier et al., 2021). This constitutive activity goes along with a relatively high constitutive β-arrestin-dependent internalization rate (Le Mercier *et al.,* 2021). Thus, binding of chemokines to GPR182 leads to their receptor-dependent internalization, and the receptor therefore functions as a chemokine scavenger. The spectrum of chemokines binding to GPR182 is broad; however, at physiological concentrations, it binds CXCL10, CXCL12 and CXCL13 (Le Mercier *et al.,* 2021). Studies in constitutive and inducible GPR182 knock-out mice have shown that the receptor plays a role in retaining hematopoietic stem cells in the bone marrow, and that a loss of GPR182 results in reduced retention of HSCs in the bone marrow (Le Mercier *et al.*, 2021).

### 1.3 RESULTS & DISCUSSION

In the present study, the inventors set out to explore a potential implication of GPR182 in the pathology and recovery of myocardial infarction (MI).

Conducting a further analysis of the global transcriptomic profiling data (obtained from single cell RNA sequencing) of an animal model of myocardial infarction made available by Tombor *et al.*, the inventors found that GPR182 expression was surprisingly upregulated in myocardial endothelial cells after myocardial infarction. In particular, GPR182 expression was found to increase within the first week after myocardial infarction in a murine model (ligation of the left anterior descending coronary artery (LAD)) (**Fig. 1**).

Next, the inventors set out to explore potential effects of an inhibition of GPR182 in a murine model of myocardial infarction. It was surprisingly and advantageously found that in mice lacking GPR182 constitutively, as well as in mice with induced endothelium-specific loss of GPR182, the infarct area was significantly reduced as compared to control animals (**Fig. 2**).

An increased intact myocardial mass was also seen on the endocardial side of the infarct area (**Fig. 3**).

Both by echocardiography as well as by magnetic resonance imaging (MRI), it was found by the inventors that GPR182-KO mice starting from the first week after the infarction showed improved cardiac parameters including an increased left ventricular ejection fraction as well as a reduced left ventricular diastolic and systolic volume compared to wild-type littermate controls (**Fig. 4**).

Loss of GPR182 had no effect on normal heart function. 3 days after myocardial infarction, the concentration of the chemokine CXCL12 in the heart tissue was significantly increased in mice lacking GPR182 (**Fig. 5**).

Thus, loss of GPR182 function leads to reduced scavenging of CXCL12 and increased levels of CXCL12. This then increases blood vessel development after myocardial infarction, resulting in decreased infarct size and improved post-infarct heart function.

In view of these results, a pharmacological inhibition of the GPR182 scavenging functions, e.g., by means of an antibody, nanobody or other agent contemplated herein, to elevate the concentration of CXCL12 in the heart will provide a novel and effective therapeutic means for the treatment of acute myocardial infarction (MI) and associated post-acute MI complications, in particular for improving post-infarct angiogenesis and/or collateral growth, resulting in improved perfusion, reduced infarct size and reduced heart failure.

When developing an agent as contemplated herein (e.g., an antagonist or inhibitor of GPR182), care has to be taken with regard to specificity. In particular, such an agent should preferably specifically inhibit the expression or activity of the GPR182 protein, without also affecting the expression or activity of other protein(s) which function(s) as signaling receptor(s) for the one or more endogenous GPR182-ligands. For example, the structurally related receptor ACKR3 (CXCR7) also functions as an atypical chemokine receptor (ACKR) for CXCL12, but, in contrast to GPR182, it induces some downstream signaling by recruitment of β-arrestin (Rajagopal *et al.,* 2010) (**Fig. 6**), and appears to have opposite functions after myocardial infarction. Endothelium-specific loss ACKR3 (CXCR7) leads to increased infarct size and reduced heart function after myocardial infarction, and cardiomyocyte-specific loss of ACKR3 (CXCR7) also reduces heart function after myocardial infarction. This is most likely due to reduced β-arrestin-mediated signaling (Hao *etal.,* 2017; Ishizuka *etal.,* 2021).

Thus, the antagonist/inhibitor should preferably be selective for GPR182 and not also block ACKR3 (CXCR7).

### Advantages of the approach compared to other therapeutic approaches

Despite the enormous progress in the therapy of myocardial infarction (MI), which is mainly due to the development of drugs and technical procedures aiming at the reperfusion of the thrombosed coronary artery, a considerable part of myocardial infarct patients still does not survive the acute myocardial infarction. In addition, those, who survive, suffer from long-term complications such as arrhythmia or heart failure. In these cases, an improvement of CXCL12-mediated vessel growth as an additional or alternative procedure should have an additive or synergistic effect on the reduction of the infarct area as well as on the risk of secondary diseases. Improving neovascularization after myocardial infarction is a novel approach, which has so far not been used in clinical therapy of acute myocardial infarction.

In contrast to systemic application of CXCL12, the blockade of the scavenger receptor GPR182 is a much more selective procedure which increases the local CXCL12 effect by just reducing the local degradation of CXCL12. This approach is therefore believed to result in a much more selective and specific CXCL12-mediated effect, which is likely to lead to less unwanted effects.

### Further contemplated indications

A recent publication confirmed the role of GPR182 as a chemokine-scavenging receptor (Torphy *et al.*, 2022). In this study, the authors showed that GPR182 is upregulated in lymphatic endothelial cells of human melanoma and that it contributes to immunotherapy resistance in cancer via scavenging of chemokines that are important for lymphocyte recruitment to tumors. In GPR182-deficient mice, it was shown that T-cell infiltration into transplanted melanomas increased and led to enhanced effector T-cell function and improved anti-tumor immunity. This was accompanied by increased intratumoral concentrations of chemokines and led to sensitization of poorly immunogenic tumors to immune checkpoint blockade and adoptive cellular therapies. It is hence plausible that an inhibition of GPR182 by agents as contemplated herein can be used to sensitize melanoma with poor immunogenicity and resistance to immune checkpoint blockade.

### Example 2: Competitive binding assay

Single cell suspensions of HEK293 cells stably expressing human or mouse GPR182 are prepared by trypsin/EDTA treatment. After inactivation of trypsin by dilution in DMEM (Gibco, 10938025) containing 10 % Fetal Bovine Serum (FBS), cells are passed through a 70 µm cell strainer to remove clumps and counted using a Neubauer chamber. Required cells are then centrifuged for 5 min at 200 g at 4°C, followed by 1 wash with binding medium (DMEM serum free, 10mM HEPES (Gibco, 15630080), 0.5 % BSA, 4°C). Then cells are incubated with defined concentration of non-labelled agent in 100 µL binding medium binding medium for 30 min at 4°C with gentle rocking to allow binding without internalization nor recycling followed by 3 washes with binding medium. Cells are then resuspended in 100 µL binding medium containing AlexaFluor-647 fluorescently labelled chemokine CXCL10, CXCL12 or CXCL13 (Almac). Each sample is then incubated in 96 well plate for 1h at 4°C with gentle rocking to allow binding without internalization nor recycling. Cells are then washed 3 times with binding medium and analyzed by flow cytometry by incubating cells with DAPI just before analysis as a counterstain for dead cells. Cells are gated in order to include only live singlets in the analysis. Mean fluorescence intensity (MFI) of the channel detecting the signal from AlexaFluor-647 fluorescently labelled chemokine is used as readout of binding intensity. Ki value of the agent is then calculated using well known standard equation in the art.

### Further References

Das S, Goldstone AB, Wang H, Farry J, D'Amato G, Paulsen MJ, Eskandari A, Hironaka CE, Phansalkar R, Sharma B, Rhee S, Shamskhou EA, Agalliu D, de Jesus Perez V, Woo YJ, Red-Horse K (2019) A Unique Collateral Artery Development Program Promotes Neonatal Heart Regeneration. Cell 176: 1128-1142 e1118
Hao H, Hu S, Chen H, Bu D, Zhu L, Xu C, Chu F, Huo X, Tang Y, Sun X, Ding BS, Liu DP, Hu S, Wang M (2017) Loss of Endothelial CXCR7 Impairs Vascular Homeostasis and Cardiac Remodeling After Myocardial Infarction: Implications for Cardiovascular Drug Discovery. Circulation 135: 1253-1264
Ishizuka M, Harada M, Nomura S, Ko T, Ikeda Y, Guo J, Bujo S, Yanagisawa-Murakami H, Satoh M, Yamada S, Kumagai H, Motozawa Y, Hara H, Fujiwara T, Sato T, Takeda N, Takeda N, Otsu K, Morita H, Toko H, Komuro I (2021) CXCR7 ameliorates myocardial infarction as a beta-arrestin-biased receptor. Sci Rep 11: 3426
Korf-Klingebiel M, Reboll MR, Grote K, Schleiner H, Wang Y, Wu X, Klede S, Mikhed Y, Bauersachs J, Klintschar M, Rudat C, Kispert A, Niessen HW, Lubke T, Dierks T, Wollert KC (2019) Heparan Sulfate-Editing Extracellular Sulfatases Enhance VEGF Bioavailability for Ischemic Heart Repair. Circ Res 125: 787-801
Kroeze WK, Sassano MF, Huang XP, Lansu K, McCorvy JD, Giguere PM, Sciaky N, Roth BL (2015) PRESTO-Tango as an open-source resource for interrogation of the druggable human GPCRome. Nat Struct Mol Biol 22: 362-369
Le Mercier A, Bonnavion R, Yu W, Alnouri MW, Ramas S, Zhang Y, Jager Y, Roquid KA, Jeong HW, Sivaraj KK, Cho H, Chen X, Strilic B, Sijmonsma T, Adams R, Schroeder T, Rieger MA, Offermanns S (2021) GPR182 is an endothelium-specific atypical chemokine receptor that maintains hematopoietic stem cell homeostasis. Proc Natl Acad Sci U S A 118
Macarthur JW, Jr., Cohen JE, McGarvey JR, Shudo Y, Patel JB, Trubelja A, Fairman AS, Edwards BB, Hung G, Hiesinger W, Goldstone AB, Atluri P, Wilensky RL, Pilla JJ, Gorman JH, 3rd, Gorman RC, Woo YJ (2014) Preclinical evaluation of the engineered stem cell chemokine stromal cell-derived factor 1alpha analog in a translational ovine myocardial infarction model. Circ Res 114: 650-659
Rajagopal S, Kim J, Ahn S, Craig S, Lam CM, Gerard NP, Gerard C, Lefkowitz RJ (2010) Beta-arrestin-but not G protein-mediated signaling by the "decoy" receptor CXCR7. Proc Natl Acad Sci U S A 107: 628-632
Saxena A, Fish JE, White MD, Yu S, Smyth JW, Shaw RM, DiMaio JM, Srivastava D (2008) Stromal cell-derived factor-1alpha is cardioprotective after myocardial infarction. Circulation 117: 2224-2231
Schmid CD, Schledzewski K, Mogler C, Waldburger N, Kalna V, Marx A, Randi AM, Geraud C, Goerdt S, Koch PS (2018) GPR182 is a novel marker for sinusoidal endothelial differentiation with distinct GPCR signaling activity in vitro. Biochem Biophys Res Commun 497: 32-38
Tombor LS, John D, Glaser SF, Luxan G, Forte E, Furtado M, Rosenthal N, Baumgarten N, Schulz MH, Wittig J, Rogg EM, Manavski Y, Fischer A, Muhly-Reinholz M, Klee K, Looso M, Selignow C, Acker T, Bibli SI, Fleming I, Patrick R, Harvey RP, Abplanalp WT, Dimmeler S (2021) Single cell sequencing reveals endothelial plasticity with transient mesenchymal activation after myocardial infarction. Nat Commun 12: 681
Torphy RJ, Sun Y, Lin R, Caffrey-Carr A, Fujiwara Y, Ho F, Miller EN, McCarter MD, Lyons TR, Schulick RD, Kedl RM, Zhu Y (2022) GPR182 limits antitumor immunity via chemokine scavenging in mouse melanoma models. Nat Commun 13: 97
Xiao L, Harrell JC, Perou CM, Dudley AC (2014) Identification of a stable molecular signature in mammary tumor endothelial cells that persists in vitro. Angiogenesis 17: 511-518
Zeymer U (2019) Herzinfarkt - Was kommt in den Jahren danach. Dtsch Arztebl 116(40): 22-26
Zhang M, Qiu L, Zhang Y, Xu D, Zheng JC, Jiang L (2017) CXCL12 enhances angiogenesis through CXCR7 activation in human umbilical vein endothelial cells. Sci Rep 7: 8289

It is understood that the definitions and embodiments as described above in the context of the first aspect of the invention also apply, in as far as possible, mutatis mutandis to the second, third, fourth, fifth and sixth aspects of the present invention.

The invention is herein described, by way of example only, with reference to the accompanying drawings for purposes of illustrative discussion of the preferred embodiments of the present invention.

Unless otherwise defined, all technical and scientific terms used herein have the same meaning as commonly understood by one of ordinary skill in the art to which this invention belongs. In case of conflict, the patent specification, including definitions, will prevail.

Regarding the embodiments characterized in this specification, in particular in the claims, it is intended that each embodiment mentioned in a dependent claim is combined with each embodiment of each claim (independent or dependent) said dependent claim depends on. For example, in case of an independent claim 1 reciting 3 alternatives A, B, and C, a dependent claim 2 reciting 3 alternatives D, E, and F and a claim 3 dependent on claims 1 and 2 and reciting 3 alternatives G, H, and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2, and 1. In case a further dependent claim 4 is present which refers to any one of claims 1 to 3, it follows that the combination of the subject-matter of claims 4 and 1, of claims 4, 2, and 1, of claims 4, 3, and 1, as well as of claims 4, 3, 2, and 1 is clearly and unambiguously disclosed.

The above considerations apply *mutatis mutandis* to all appended claims.

The entirety of each patent, patent application, publication and document referenced herein hereby is incorporated by reference. Citation of the above patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

Modifications may be made to the foregoing without departing from the basic aspects of the technology. Although the technology has been described in substantial detail with reference to one or more specific embodiments, those of ordinary' skill in the art will recognize that changes may be made to the embodiments specifically disclosed in this application, yet these modifications and improvements are within the scope and spirit of the technology.

The technology illustratively described herein suitably may be practiced in the absence of any element(s) not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the technology claimed. The terms "method" and "process" are used interchangeably herein. The term "a" or "an" can refer to one of or a plurality of the elements it modifies (e.g., "a cell" can mean "one or more cells") unless it is contextually clear either one of the elements or more than one of the elements is described.

The term "about" as used herein refers to a value within 10% of the underlying parameter (i.e., plus or minus 10%), and a use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). It should hence be understood that, although the present technology has been specifically disclosed by representative embodiments and optional features, modification and variation of the concepts herein disclosed may be resorted to by those skilled in the art, and such modifications and variations are considered within the scope of this technology.

Certain embodiments of the technology are set forth in the claim(s) that follow(s).

## Claims

1. An agent that inhibits the expression or activity of the G Protein-Coupled Receptor 182 (GPR182) protein for use in the treatment or prevention of a pathological condition selected from myocardial infarction, myocardial ischemia, myocardial necrosis, cardiac hypertrophy, cardiac fibrosis, limb ischemia, ischemia-related tissue degeneration, stroke and a cancer,
wherein
(a) the agent that inhibits the expression of the GPR182 protein is selected from an siRNA, an miRNA, an shRNA, a ribozyme and an antisense nucleic acid molecule; and/or
(b) the agent that inhibits the activity of the GPR182 protein specifically binds to the GPR182 protein and inhibits binding of one or more endogenous ligands to the GPR182 protein, and wherein the agent is selected from an antibody, an Fc fusion polypeptide, an adnectin, an affibody, an affilin, an anticalin, an atrimer, an avimer, an evibody, a Kunitz-type domain, a designed ankyrin repeat protein (DARPin), a fynomer, a peptide or peptidomimetic, an aptamer, and a small molecule; or any combination and/or hetero- or homo-oligomeric, covalent or non-covalent complex thereof.

2. The agent for use according to claim 1(b), wherein the binding of the agent to the GPR182 protein inhibits the GPR182-mediated internalization of the one or more endogenous GPR182-ligands.

3. The agent for use according to claim 1(b) or 2, wherein the one or more endogenous ligands are one or more C-X-C chemokine ligand(s), more preferably one or more of C-X-C motif chemokine ligand 10 (CXCL10), C-X-C motif chemokine ligand 12 (CXCL12) and/or C-X-C motif chemokine ligand 13 (CXCL13).

4. The agent for use according to any one of claims 1(b) to 3, wherein the agent specifically binds to one or more epitopes within one or more of the extracellular portions of the GPR182 protein; wherein preferably the one or more epitopes are comprised in:
(i) the extracellular portion of the N-terminal domain; and/or
(ii) the second extracellular loop (ECL2)
of the GPR182 protein.

5. The agent for use according to any one of claims 1(b) to 4, wherein the agent:
(i) exhibits a higher binding affinity, preferably an at least 1.5-fold, 2-fold, 3-fold, 4-fold, 5-fold, 6-fold, 7-fold, 8-fold, 9-fold, 10-fold or higher binding affinity, towards the GPR182 protein as compared to the binding affinity between the one or more endogenous GPR182-ligands and the GPR182 protein; and/or
(ii) binds to the GPR182 protein with a dissociation constant (K_{D}) which is equal to or less than 10⁻⁶ M, 10⁻⁷ M or 10⁻⁸ M, preferably less than 41 nM, more preferably less than 10⁻⁹ M or 10⁻¹⁰ M;

6. The agent for use according to any one of claims 1 to 5, wherein the agent is to be administered in combination, or sequentially, with one or more of:
(i) a thrombolytic drug; preferably selected from:
- tissue plasminogen activator; preferably alteplase, retaplase, and/or tenecteplase;
- streptokinase;
- anistreplase; and
- urokinase;
(ii) an anticoagulant; preferably selected from:
- a heparin; preferably unfractionated heparin (UFH) and/or low molecular weight heparin (LMWH);
- a factor Xa inhibitor; preferably apixaban and/or rivaroxaban;
and
- hirudine and/or bivalirudin;
(iii) a platelet agglutination inhibitor; preferably selected from:
- an irreversible cyclooxygenase inhibitor; preferably acetylsalicylic acid (ASA) and/or triflusal;
- an adenosine diphosphate (ADP) receptor inhibitor; preferably cangrelor, clopidogrel, pasugrel, ticagrelor and/or ticlopidine;
- a phosphodiesterase inhibitor; preferably cilostazol;
- a protease-activated receptor-1 (PAR-1) antagonist; preferably vorapaxar,
- a glycoprotein IIB/IIIA inhibitor; preferably abciximab, eptifibatide, and/or tirofiban;
- an adenosine reuptake inhibitor; preferably dipyridamole;
- a thromboxane inhibitor;
- a thromboxane synthase inhibitor;
- a thromboxane receptor antagonist; preferably terutroban;
(iv) a beta blocker; preferably selected from propranolol, alprenolol, bucindolol, carteolol, carvedilol, labetalol, levobunolol, medroxalol, mepindolol, metipranolol, nadolol, oxprenolol, penbutolol, pindolol, sotalol, timolol, acebutolol, atenolol, betaxolol, bisoprolol, celiprolol, esmolol, metoprolol, and nebivolol; and
(v) an angiotensin-converting-enzyme inhibitor (ACE inhibitor); preferably selected from benazepril, zofenopril, perindopril, trandolapril, captopril, enalapril, lisinopril, and ramipril;
(vi) a nitrate; preferably isosorbide dinitrate, isosorbide mononitrate and/or pentaerythritol tetranitrate;
(vii) oxygen;
(viii) one or more C-X-C chemokine receptor type 4 (CXCR4) agonist(s), preferably CXCL12 and/or one or more synthetic CXCR4 agonist(s);
(ix) one or more C-X-C motif chemokine receptor type 7 (CXCR7) agonist(s), preferably CXCL11, CXCL12 and/or one or more synthetic CXCR7 agonist(s);
(x) a C-X-C motif chemokine ligand 10 (CXCL10); and/or
(xi) a C-X-C motif chemokine ligand 13 (CXCL13).

7. The agent for use according to any one of claims 1 to 5, wherein the agent is to be administered in combination, or sequentially, with one or more of:
(i) one or more chemotherapeutic agent(s);
(ii) an adoptive cellular therapy, preferably an adoptive T cell therapy;
(iii) one or more immune-checkpoint-inhibitors, preferably selected from an antibody directed against cytotoxic T-lymphocyte-associated antigen 4 (CTLA4), programmed death 1 (PD-1), programmed death ligand 1 (PD-L1) and programmed death ligand 2 (PD-L2) or combinations thereof; and/or
(iv) one or more C-X-C motif chemokine receptor type 3 (CXCR3) agonist(s), preferably CXCL9, CXCL10, or CXCL11 and/or one or more synthetic CXCR7 agonist(s).

8. The agent for use according to any one of claims 1(a) to 6, wherein the agent is a (poly)peptide, and is to be administered in the form of a polynucleotide encoding said (poly)peptide;
wherein preferably the polynucleotide is:
(i) comprised in a vector; preferably in a viral vector, more preferably in an adeno-associated virus (AAV) vector; or
(ii) an RNA, preferably an mRNA;

9. The agent for use according to any one of claims 1 to 8, wherein the agent does not possess substantial cytotoxic and/or immunogenic activity.

10. The agent for use according to any one of claims 1(a) to 9, wherein the agent is an antibody or Fc fusion polypeptide and does not cause substantial antibody-dependent cellular cytotoxicity (ADCC) and/or complement-dependent cytotoxicity (CDC).

11. The agent as defined in any one of the preceding claims for use in:
(i) promoting tissue repair, preferably angiogenesis and/or vascularisation, after (acute) myocardial infarction;
(ii) reducing infarction size and/or volume after (acute) myocardial infarction;
(iii) enhancing the perfusion and/or reducing the risk for or severity of heart insufficiency, myocardial necrosis, cardiac hypertrophy, cardiac fibrosis, limb ischemia, ischemia-related tissue degeneration, and/or ventricular arrhythmias after (acute) myocardial infarction;
(iv) promoting angiogenesis and/or vascularisation in an ischemic disease;
(v) enhancing anti-tumor immunity;
(vi) enhancing the tumor-infiltration by T cells, preferably CD8+ cytotoxic T cells and/or CAR T cells; and/or
(vii) enhancing the efficacy of adoptive cellular therapy.

12. The agent for use according to any one of claims 1(a) to 11, wherein the agent is
(i) distinct from CXCL12, CXCL10, and/or CXCL13; and/or
(ii) heterologous to the subject which is to be administered with the agent.

13. An *in vitro* method for identifying an agent suitable for the use according to any one of claims 1 to 12, comprising assessing, in the presence of a candidate agent, the binding of one or more endogenous GPR182-ligands to cells expressing the GPR182 protein, wherein a reduced binding of the one or more endogenous GPR182-ligands in the presence of the candidate agent as compared to in the absence of the candidate ligand indicates that the candidate agent is suitable for the use according to any one of claims 1 to 12.

14. A method for evaluating the efficacy of an agent as defined in any of the preceding claims in the treatment of a myocardial infarction, comprising assessing before, and/or simultaneously with, and subsequent to an administration of the agent one or more of the following:
(a) the size or volume of the infarction area;
(b) the left ventricular ejection fraction and/or left ventricular end-diastolic volume, and/or left ventricular end-systolic volume;
(c) the level of CXCL12 in the heart and/or blood; and/or
(d) the plasma level(s) of one or more biological markers of myocardial damage, wherein the biological marker is preferably troponin-I and/or troponin-T;
whereby a decrease of the volume of the infarction area, an improvement of the left ventricular ejection fraction, a reduction of the left ventricular end-diastolic volume, a reduction of the left ventricular end-systolic volume, an increase of the CXCL12 concentration in the heart and/or blood, and/or a decrease of the plasma level(s) of the one or more biological markers of myocardial damage determined subsequent to the administration of the agent provides an indication of the agent being efficacious in the treatment of myocardial infarction.

15. A method for evaluating the efficacy of an agent as defined in any of the preceding claims in the treatment of a cancer, comprising assessing before, and/or simultaneously with, and subsequent to an administration of the agent to a subject one or more of the following:
(a) the level of CXCL9 and/or CXCL10 in the cancer and/or blood;
(b) the level of T cells, preferably CD8+ cytotoxic T cells, in the cancer and/or blood; and/or
(c) the size of the cancer;
whereby an increase of the CXCL9 and/or CXCL10 concentration in the cancer and/or the blood, and/or an increase of the level of T cells, preferably CD8+ cytotoxic T cells, in the cancer and or blood, and/or a decrease of the size of the cancer determined subsequent to the administration of the agent provides an indication of the agent being efficacious in the treatment of the cancer.
